# EUROPEAN PATENT APPLICATION

(11) **EP 4 386 004 A1**
(43) Date of publication of application: **19.06.2024**
(21) Application number: 22855496.0
(22) Date of filing: 11.08.2022
(51) Int. Cl.: C07K 16/28, C07K 16/22, C12N 15/13, A61K 39/395

(54) **ANTI-VEGF A AND -VEGF C BISPECIFIC ANTIBODY AND USE THEREOF**

(30) Priority: 13.08.2021 CN 202110932078
(71) Applicant: Innovent Biologics (Suzhou) Co., Ltd., Suzhou, Jiangsu 215123 (CN)
(72) Inventor: LI, Yiming, Suzhou, Jiangsu 215123 (CN); HU, Siyi, Suzhou, Jiangsu 215123 (CN); CHEN, Bingliang, Suzhou, Jiangsu 215123 (CN); ZHOU, Shuaixiang, Suzhou, Jiangsu 215123 (CN)
(74) Representative: Sagittarius IP
(86) International application number: PCT/CN2022/111717
(87) International publication number: WO 2023/016516

(57) **Abstract**

A single-domain antibody polypeptide specifically binding to VEGF C and a construct thereof, an anti-VEGF C/VEGF A bispecific binding protein, polynucleotides encoding the polypeptide and protein, an expression vector, a host cell, a pharmaceutical composition thereof, and a method and use for treating diseases related to neovascularization.

## Description

### TECHNICAL FIELD

The present invention relates to a single-domain antibody polypeptide specifically binding to VEGF C and a construct thereof, particularly an anti-VEGF C/VEGF A bispecific binding protein. The present invention also relates to a polynucleotide encoding the polypeptide and protein, an expression vector, a host cell, a pharmaceutical composition thereof, and a method and use for treating a neovascularization-associated disease.

### BACKGROUND

Vascular endothelial growth factor (VEGF) is a major regulatory factor of vascular development as well as blood and lymphatic vessel function in adults during health and disease. Currently, it is known that the VEGF family consists of five structurally related factors: VEGFA (also known as VEGFA165), VEGFB, VEGFC, VEGFD, and placental growth factor (PIGF). VEGF family members are primarily present in the form of a homodimeric polypeptide, which induce signaling and elicit corresponding biological effects by binding to related VEGF receptors.

Vascular endothelial growth factor A (VEGF-A), as an angiogenic cytokine, is involved in normal and abnormal angiogenic processes by interacting with two high-affinity transmembrane tyrosine kinase receptors (VEGFR-1 and VEGFR-2). Currently, a number of different approaches have been proposed to block the VEGF-A pathway, thereby improving neovascularization-associated diseases. VEGF-A blockers/antagonists that have been proposed include: neutralizing antibodies targeting VEGF-A as well as soluble decoy receptors and Trap molecules that prevent VEGF-A from binding to its normal receptors. For example, a humanized monoclonal anti-VEGF-A antibody bevacizumab (trade name Avastin) has been approved for the treatment of colorectal cancer, breast cancer, and lung cancer. Ranibizumab (trade name Lucentis) is a monoclonal antibody fragment derived from the same parent murine antibody as bevacizumab which is much smaller than the parent molecule and has undergone affinity maturation to provide stronger VEGF-A binding properties (WO98/45331). Ranibizumab has been approved for the treatment of wet age-related macular degeneration. Aflibercept (VEGFA-Trap, trade name Elyea) is a recombinant fusion protein formed by fusing ligand-binding domains from human VEGF receptors 1 and 2 to an Fc region of human IgG1. Aflibercept has been approved for the treatment of neovascularization-associated retinal diseases such as age-related macular degeneration, and is in clinical trials for the treatment of solid tumors.

Vascular endothelial growth factor C (VEGF-C) is identified as a lymphangiogenic cytokine and acts through tyrosine kinase receptors VEGFR2 and VEGFR3. VEGF-C is involved in the process of neovascularization by binding to VEGFR2 on vascular endothelial cells. In addition, VEGF-C stimulates lymphangiogenesis and lymphatic endothelial cell growth and migration by binding to the receptor VEGFR3. VEGFR3, although structurally similar to VEGFR1 and VEGFR2, does not bind to VEGF-A. It has been found that VEGFR3 is highly expressed in vascular endothelial cells in addition to lymphatic vessel cells. It has been proposed that blocking the VEGF-C/VEGFR3 signaling pathway can be used to inhibit lymphangiogenesis and metastasis of tumors in a variety of metastatic tumor models. In addition, VEGF-C Trap molecules, such as OPT-032 (a VEGFC/D inhibitor constructed from the extracellular domain of the ligand of VEGFR3) developed by Opthea Limited, have been developed for the treatment of neovascularization-associated retinopathies.

The involvement of neovascularization has been identified in a number of disease processes including, for example, cancers, autoimmunity, retinopathies, and the like. In view of the role of VEGF-A and VEGF-C in vascular and lymphatic vessel growth, it has been proposed that VEGF-A and VEGF-C may have synergistic promotion effects on each other in neovascularization-associated diseases such as in the growth and metastasis of tumors. Thus, there is a need in the art to develop a new anti-neovascularization molecule, particularly a bispecific molecule capable of targeting both VEGF-A and VEGF-C. Such bispecific molecules would help block vascular endothelial growth factors A and C simultaneously to arrest the progression of neovascularization-associated diseases. Such bispecific molecule would also provide advantages in administration over the combination of individual anti-VEGF-A molecules and anti-VEGF-C molecules. Currently, in the treatment of ocular diseases, a variety of individual anti-VEGF-A molecules such as aflibercept, ranibizumab and conbercept show limitations, that is, as injection formulations, they need to be administered intravitreally, which is inconvenient and also results in poor patient compliance and a large treatment burden. Therefore, it would be advantageous to design a bispecific molecule that can replace this combination to potently and specifically bind to and neutralize both VEGF-A and VEGF-C.

### SUMMARY

In order to meet the needs described above, the inventors have conducted intensive studies to provide a novel high-affinity anti-VEGF-C single-domain antibody (scAb) polypeptide through humanization and affinity maturation based on screening of phage display libraries; moreover, the single-domain antibody polypeptide is used as a component and combined with an anti-VEGF-A molecule to construct a bispecific molecule having excellent dual VEGF-A and VEGF-C antagonistic activity and an anti-neovascularization effect.

Accordingly, in one aspect, the present invention provides a single-domain antibody (sdAb) specifically binding to human VEGF-C. In one embodiment, the anti-VEGF-C single-domain antibody of the present invention comprises a VHH domain having the following formula: FR1-CDR1-FR2-CDR2-FR3-CDR3-FR4, wherein the CDR1-3 comprise or consist of amino acid sequences selected from:
(1) amino acid sequences set forth in SEQ ID NOs: 1, 2, and 3;
(2) amino acid sequences set forth in SEQ ID NOs: 9, 10, and 11;
(3) amino acid sequences set forth in SEQ ID NOs: 17, 18, and 19; and
(4) amino acid sequences set forth in SEQ ID NOs: 21, 22, and 23.

In a further aspect, the present invention provides a protein comprising at least the single-domain antibody of the present invention. Preferably, the protein is a fusion protein or a chimeric polypeptide, such as a VHH-Fc antibody. In a further aspect, the present invention provides a bispecific binding protein comprising
(i) a first antigen-binding component specifically binding to human VEGF C; and
(ii) a second antigen-binding component specifically binding to human VEGF A,

wherein the first antigen-binding component comprises the single-domain antibody polypeptide of the present invention; and
the bispecific binding protein has dual antagonistic activity, inhibiting the binding of VEGF A to its VEGF receptor and inhibiting the binding of VEGF C to its VEGF receptor.

In a further aspect, the present invention provides a polynucleotide encoding the molecule (single-domain antibody polypeptide, protein, or bispecific binding protein) of the present invention, a vector, a host cell, and a pharmaceutical composition, a pharmaceutical combination, and a kit comprising the molecule of the present invention.

In a further aspect, the present invention provides use of the molecule (single-domain antibody polypeptide, protein, or bispecific binding protein) of the present invention in the prevention and/or treatment of a disease, wherein the disease is, for example, a neovascularization-associated disease, such as a tumor and an ocular disease.

In a further aspect, the present invention further provides diagnostic use of the molecule (single-domain antibody polypeptide, protein, or bispecific binding protein) of the present invention.

### BRIEF DESCRIPTION OF THE DRAWINGS

FIGs. 1A-1D show ELISA assays in which anti-VEGFC VHH antibodies block the binding of VEGFC to receptors VEGFR2/VEGFR3. A: VEGFR2 blocking assay of anti-VEGFC VHH; B: VEGFR3 blocking assay of anti-VEGFC VHH; C: VEGFR3 blocking assay of anti-VEGFC humanized VHH; D: VEGFR3 blocking assay of anti-VEGFC affinity-matured VHH.
FIG. 2 shows an assay in which the anti-VEGFCVHH antibodies block VEGFC-induced HEK 293 KDR reporter activation.
FIG. 3 shows an assay in which the anti-VEGFCVHH antibodies block VEGFC-induced Baf3-FLT4 proliferation.
FIGs. 4A-4B show assays in which the humanized anti-VEGFC VHH antibodies block VEGFC-induced HEK 293 KDR reporter activation.
FIGs. 5A-5B show assays in which the humanized anti-VEGFC VHH antibodies block VEGFC-induced Baf3-FLT4 proliferation.
FIGs. 6A-6B show assays in which the affinity-matured molecules, anti-VEGFC VHH antibodies, block VEGFC-induced HEK 293 KDR reporter activation.
FIGs. 7A-7B show assays in which the affinity-matured anti-VEGFC VHH antibodies block VEGF-C-induced Baf3-FLT4 proliferation.
FIG. 8 shows assays in which bispecific antibody molecules block VEGFA-induced HEK 293 KD reporter activation.
FIG. 9 shows assays in which bispecific antibody molecules block VEGFC-induced HEK 293 KDR reporter activation.
FIGs. 10A-10C show assays in which bispecific antibody molecules inhibit VEGFC-induced BaF3-FLT4 proliferation.
FIGs. 11A-11B show assays in which bispecific antibody molecules inhibit VEGFA+C-induced HUVEC proliferation.
FIGs. 12A-12B show assays in which bispecific antibody molecules inhibit VEGFA+C-induced HUVEC tube formation; A: tube formation images; B: statistical results of tube formation.
FIGs. 13A-13C show assays in which bispecific antibody molecules inhibit neovascularization in an A375 subcutaneous tumor model; A: A375 tumor mass statistics; B: A375 tumor volume statistics; C: CD31 staining images of the A375 tumor.
FIG. 14 shows assays in which bispecific antibody molecules inhibit laser-induced CNV
FIG. 15 shows fundus angiography images of anti-VEGFA/VEGF C bispecific antibodies inhibiting laser-induced choroidal neovascularization.
FIG. 16 shows OCT images of anti-VEGFA/VEGF C bispecific antibodies inhibiting laser-induced retinal thickening.
FIGs. 17A-17B show that anti-VEGFA/VEGF C bispecific antibodies inhibit laser-induced pathological changes in the choroidal neovascularization model.
FIGs. 18A-18B show structural schematic diagrams of two bispecific antibodies constructed using a VEGF-A binding domain-Fc fusion protein and an anti-VEGF-A Fab antibody in combination with an anti-VEGF-C VHH antibody as a component.
FIG. 19 shows CDR1-3 sequences and VHH sequences of anti-VEGF-C VHH single-domain antibodies of the present invention.

### DETAILED DESCRIPTION

Unless otherwise defined, all technical and scientific terms used herein have the same meaning as commonly understood by those of ordinary skill in the art to which the present invention belongs. All publications, patent applications, patents, and other references mentioned herein are incorporated by reference in their entireties. In addition, the materials, methods, and examples described herein are illustrative only and are not intended to be limiting. Other features, objectives, and advantages of the present invention will be apparent from the specification and drawings, and from the appended claims.

### Definitions

The term "about" used in combination with a numerical value is intended to encompass the numerical values in a range from a lower limit less than the specified numerical value by 5% to an upper limit greater than the specified numerical value by 5%.

As used herein, the term "comprise" or "include" is intended to mean that the elements, integers, or steps are included, but not to the exclusion of any other elements, integers, or steps.

The term "antibody" is used herein in the broadest sense, refers to a protein comprising an antigen-binding site, and encompasses natural and artificial antibodies with various structures, including but not limited to monoclonal antibodies, polyclonal antibodies, multispecific antibodies (e.g., bispecific antibodies), single-chain antibodies, single-domain antibodies, full-length antibodies, and antibody fragments.

The terms "whole antibody" and "full-length antibody" are used interchangeably herein to refer to an antibody molecule having the structure of a native immunoglobulin molecule. In the case of a conventional four-chain IgG antibody, the full-length antibody comprises two heavy chains (H) and two light chains (L) interconnected by disulfide bonds. In the case of a heavy chain antibody having only heavy chains but lacking light chains, the full-length antibody comprises two heavy chains (H) interconnected by disulfide bonds.

For the conventional four-chain IgG antibody, the full-length antibody heavy chain generally consists of a heavy chain variable region (abbreviated herein as VH) and a heavy chain constant region, wherein the heavy chain constant region comprises at least 3 domains CH1, CH2, and CH3. The full-length antibody light chain consists of a light chain variable region (abbreviated herein as VL) and a light chain constant region, wherein the light chain constant region consists of one domain CL. Each heavy chain variable region VH or each light chain variable region consists of three CDRs and 4 FRs, arranged from amino-terminus to carboxyl-terminus in the following order: FR1, CDR1, FR2, CDR2, FR3, CDR3, and FR4.

For the heavy chain antibody, the full-length antibody heavy chain generally consists of a heavy chain variable region (abbreviated herein as VHH) and a heavy chain constant region. The heavy chain constant region consists of domains CH2 and CH3. Each VHH consists of three CDRs and four FRs, arranged from amino-terminus to carboxyl-terminus in the following order: FR1, CDR1, FR2, CDR2, FR3, CDR3, and FR4.

The term "antigen-binding fragment" of an antibody is a portion or segment of a whole antibody or a full-length antibody that has fewer amino acid residues than the whole antibody or the full-length antibody, but is capable of binding to an antigen or competes with the full-length antibody (i.e., a full-length antibody from which the antigen-binding fragment is derived) for binding to an antigen. The antigen-binding fragment may be prepared by recombinant DNA techniques, or by enzymatic or chemical cleavage of an intact antibody. The antigen-binding fragment includes, but is not limited to, a Fab, a Fab', a F(ab')2, an Fv, a single-chain Fv, a diabody, a single-domain antibody (sdAb), and a nanobody. For example, a Fab fragment can be obtained by papain digestion of a full-length antibody. In addition, the F(ab')2, a dimer of the Fab', is a bivalent antibody fragment produced by pepsin digestion of a portion below disulfide bonds in a hinge region of a complete antibody. The F(ab')2 can be reduced by disrupting the disulfide bonds in the hinge region under neutral conditions, and the F(ab')2 dimer is thus converted into Fab' monomers. The Fab' monomer is essentially a Fab fragment with a hinge region. The Fv fragment comprises the VL and VH domains of a single arm of an antibody. The two domains VL and VH of the Fv fragment can be encoded by separate genes, but the two domains can also be linked, using recombinant methods, by a synthetic linker peptide so that they are produced as a single protein chain in which the VL and VH regions pair to form a single-chain Fv (scFv).

The term "variable region" or "variable domain" of an antibody refers to a domain of a heavy chain or light chain of the antibody involved in the binding of the antibody to an antigen. In the case of the heavy chain antibody, for example, a heavy chain antibody from Camelidae, a single VH domain may be sufficient to provide antigen-binding specificity. VHH of a native heavy chain antibody has a similar structure to a heavy chain variable region VH of a native IgG antibody, i.e., it comprises four conserved framework regions (FRs) and three complementarity determining regions (CDRs), and has a structure of FR1-CDR1-FR2-CDR2-FR3-CDR3-FR4.

"Complementarity determining region" or "CDR region" or "CDR" or "highly variable region" of an antibody is a region in an antibody variable domain (VH or VHH) that is highly variable in sequence, forms a structurally defined loop ("a hypervariable loop") and/or comprises antigen-contacting residues ("antigen-contacting sites"). CDRs are primarily responsible for binding to antigen epitopes. The CDRs of heavy and light chains are numbered sequentially from the N-terminus and are generally referred to as CDR1, CDR2, and CDR3. The CDRs located in a heavy chain variable domain of an antibody are also referred to as HCDR1, HCDR2, and HCDR3, whereas the CDRs located in a light chain variable domain of an antibody are referred to as LCDR1, LCDR2, and LCDR3. In a given amino acid sequence of a light chain variable region or a heavy chain variable region, the CDR sequence thereof may be determined using a variety of schemes well known in the art, for example, Chothia based on the three-dimensional structure of antibodies and the topology of the CDR loops, Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (the international ImMunoGeneTics information system, imgt.cines.fr/ on the World Wide Web), and North CDR definition based on the affinity propagation clustering using a large number of crystal structures (North et al., "A New Clustering of Antibody CDR Loop Conformations", Journal of Molecular Biology, 406, 228-256, (2011)).

The following are the regional ranges of the CDRs defined using the kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used herein encompasses CDR sequences determined by any one of the schemes described above.

CDRs can also be determined based on having the same Kabat numbering positions as the reference CDR sequences.

Unless otherwise stated, residue positions of an antibody variable region (including heavy chain variable region residues and light chain variable region residues) in the present invention are positions numbered according to the Kabat numbering system (Kabat et al., Sequences of Proteins of Immunological Interest, 5th Ed. Public Health Service, National Institutes of Health, Bethesda, Md. (1991)).

The term "Fc domain" or "Fc region" is used herein to define a C-terminus region of an immunoglobulin heavy chain, which comprises at least a portion of a constant region. The term includes Fc regions of native sequences and variant Fc regions. A native immunoglobulin "Fc domain" comprises two or three constant domains, i.e., a CH2 domain, a CH3 domain, and an optional CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies. Unless otherwise stated herein, amino acid residues in the Fc region or heavy chain constant region are numbered according to the EU numbering system (also known as the EU Index) as described in, for example, Kabat et al., Sequences of Proteins of Immunological Interest, 5th Ed., Public Health Service, National Institutes of Health, Bethesda, MD, 1991. Herein, the terms "Fc region", "Fc portion", and "Fc fragment" do not comprise a heavy chain variable region VH and a light chain variable region VL as well as a heavy chain constant region CH1 and a light chain constant region CL of an immunoglobulin, but in some cases, they can comprise a hinge region at the N-terminus of the heavy chain constant region.

The term "chimeric antibody" is an antibody molecule in which: (a) a constant region or a portion thereof is modified, substituted, or exchanged such that antigen-binding sites are linked to constant regions of different or modified classes, effector functions, and/or species sources, or disparate molecules imparting new properties (e.g., enzymes, toxins, hormones, growth factors, and drugs) to chimeric antibodies, etc.; or (b) a constant region or a portion thereof is modified, substituted, or exchanged by variable regions with different or modified antigen-binding specificities. For example, a Camelidae heavy chain antibody may be modified by substituting its constant region with a constant region from a human immunoglobulin. Due to the substitution with a human constant region, the chimeric antibody may retain its specificity for recognizing antigens, while having reduced antigenicity in humans as compared to the original Camelidae antibody.

As used herein, the "humanized antibody" is an antibody that retains the antigen-specific reactivity of a non-human antibody (such as an alpaca monoclonal antibody) and has lower immunogenicity when administered to humans as a therapeutic agent. This can be achieved, for example, by retaining non-human antigen-binding sites and substituting the remainder of the antibodies with their human counterparts (i.e., the portions of the constant and variable regions not involved in binding are substituted with the corresponding parts of human antibodies).

As used herein, the terms "fusion protein" and "chimeric polypeptide" are used interchangeably and refer to a larger polypeptide formed by fusing at least two heterologous polypeptide sequences, optionally, via a linker. The fusion protein may be produced by recombinant expression.

The term "heterologous" in reference to a fusion protein or chimeric polypeptide means that the fusion protein or chimeric polypeptide comprises two or more subsequences that are not present in the same protein or polypeptide in nature.

The term "recombinant" refers herein to the production of a polypeptide or protein by a biological host. The host may be selected from, but is not limited to, mammalian expression systems, insect cell expression systems, yeast expression systems, and bacterial expression systems. In one embodiment, the polypeptide/protein of the present invention is a recombinant polypeptide/protein produced by the expression in a prokaryotic (e.g., an *E*. *coli* cell) or eukaryotic host cell (e.g., a mammalian host cell).

As used herein, the term "monospecific" refers to a polypeptide/protein molecule having one or more antigen-binding sites, each of which binds to the same epitope of the same antigen. As used herein, the term "multispecific" refers to a polypeptide/protein molecule having at least two antigen-binding sites that bind to different epitopes (different epitopes on the same antigen or different epitopes on different antigens). In some embodiments, the present invention provides a monospecific binding molecule comprising the single-domain antibody of the present invention. In some embodiments, the molecule is monovalent, such as a single VHH-Fc polypeptide having only a single VHH domain. In other embodiments, the molecule is multivalent, such as a heavy chain antibody formed by the dimerization of two VHH-Fc polypeptides through the Fc regions. In some embodiments, the present invention provides a bispecific binding molecule, two antigen-binding specificities of which are directed against antigens VEGF-A and VEGF-C, particularly human VEGFA and human VEGFC, respectively, wherein preferably, at least one anti-VEGF-C specificity is provided by the single-domain antibody of the present invention.

The terms "antigen-binding site" and "antigen-binding domain" as used herein are used interchangeably and refer to a region in a molecule that actually binds to an antigen of interest. Examples of the antigen-binding site include, for example, but are not limited to, variable domains of antibodies, extracellular ligand-binding domains of receptors, and the like. Preferably, the VEGFC antigen-binding site for use in the bispecific binding protein of the present invention is provided by a variable domain (i.e., "VHH") of an anti-VEGFC heavy chain antibody; the VEGFA antigen-binding site for use in the bispecific binding protein of the present invention is provided by the paired heavy chain variable regions (VHs) and light chain variable regions (VLs) of an anti-VEGFA antibody, or by a VEGF receptor extracellular domain polypeptide fragment that specifically binds to VEGFA, or by an artificial ligand "Trap" molecule.

As used herein, the term ligand "Trap" molecule is a fusion protein comprising a ligand-interacting extracellular domain of a receptor and a human IgG Fc region. A variety of trap molecules that "trap" VEGF ligands, including VEGFA and VEGFC, have been developed. These Trap molecules may be used to bind to the corresponding ligands in the extracellular environment and reduce their concentration. In one embodiment, the anti-VEGFA component of the present invention comprises a trap molecule in the form of an Fc fusion protein that "traps" VEGFA. The trap molecule is capable of competing with the native VEGFA cellular receptor for binding to VEGFA, thereby inhibiting VEGFA-induced signaling. In one embodiment, the trap molecule in the form of an Fc fusion protein comprises an amino acid sequence set forth in SEQ ID NO: 25. The VEGFA-trap molecule is formed by fusing extracellular ligand-binding domains from VEGF receptors 1 and 2 to an Fc portion of human IgG1, with amino acids entirely derived from human, thereby minimizing the immunogenicity of the molecule in humans.

As used herein, the term "VEGFA" or "VEGF-A" refers to vascular endothelial growth factor A (e.g., the human VEGFA protein under accession number UniProt P15692). VEGFA binds to receptors VEGFR1 (also known as FLT1) and VEGFR2 (also known as KDR). Herein, "antigen-binding specificity against VEGFA" refers to a binding site or binding domain in a molecule that specifically binds to human VEGFA.

The term "VEGFC" or "VEGF-C" refers to vascular endothelial growth factor C (e.g., the human VEGFC protein under accession number UniProt P49767). VEGFC binds to receptors VEGFR2 (also known as KDR) and VEGFR3 (also known as FLT4). Herein, "antigen-binding specificity against VEGFC" refers to a binding site or binding domain in a molecule that specifically binds to human VEGFC. In one embodiment, the VHH antigen-binding site of the molecule of the present invention that binds to VEGFC has high-affinity binding activity for human VEGFC, for example, with a monovalent binding affinity KD value of about 1-10 nM, or a bivalent binding affinity of about 0.1-0.7 nM, as measured by bio-layer interferometry.

"Anti-VEGF-C component" refers herein to a polypeptide capable of binding to the VEGF-C protein. For example, the anti-VEGF-C component may be a polypeptide comprising the VHH domain of the present invention. In one embodiment, the anti-VEGF-C component is a single-domain antibody binding to VEGF-C. In yet another embodiment, the anti-VEGF-C component comprises an amino acid sequence having at least 80%, 85%, 90%, 91%, 92%, 93%, 94%, 95%, 96%, 97%, 98%, 99%, or about 100% identity to an amino acid sequence selected from SEQ ID NOs: 4, 8, 12, 16, 20, and 24. Preferably, the anti-VEGF-C component comprises 3 CDR sequences of the VHH domain having the amino acid sequences selected from SEQ ID NOs: 4, 8, 12, 16, 20, and 24, wherein the CDR sequences are defined according to the Kabat, or defined according to Chothia; or wherein the CDR1 is defined according to a combination of Kabat and Chothia, and the CDR2 and CDR3 are defined according to Kabat.

"Anti-VEGF-A component" refers herein to a polypeptide capable of binding to the VEGF-A protein. For example, an anti-VEGF-A component may be a chimeric polypeptide/fusion protein comprising a VEGFA receptor extracellular domain polypeptide and an Fc portion fused to its C-terminus. The anti-VEGF-A component may also be an anti-VEGFA antibody comprising a heavy chain and a light chain, such as a Fab antibody or a fragment of other full-length antibodies. In one embodiment, the anti-VEGF-A component is provided by a trap molecule in the form of an Fc fusion protein and forms a dimer through the dimerization of the Fc regions. In another embodiment, the anti-VEGF-A component is provided by an anti-VEGFA antibody in the form of a Fab, wherein a heavy chain of the Fab (i.e., a polypeptide chain comprising a heavy chain constant region) and a light chain of the Fab (i.e., a polypeptide chain comprising a light chain constant region) are paired to form a dimer.

The "Fab fragment", "Fab", and "Fab antibody" are used interchangeably herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains are paired to form an antigen-binding site. As used herein, Fab is also referred to as crossFab when one polypeptide chain of Fab comprises VH linked to CL and the other polypeptide chain comprises VL linked to CH1.

Herein, a Fab polypeptide chain comprising a heavy chain constant region CH1 is also referred to as a Fab heavy chain; correspondingly, a Fab polypeptide chain comprising a light chain constant region CL is also referred to as a Fab light chain. Thus, for a Fab antibody, CH1 may be linked to VH or CL in the Fab heavy chain, and CL may be linked to VL or VH in the Fab light chain, provided that the VH and VL are capable of being paired to form an antigen-binding site specifically binding to an antigen of interest.

The term "VHH domain" is used herein to refer to a heavy chain variable domain derived from a heavy chain antibody lacking a light chain (also sometimes referred to herein as an HcAb antibody), also referred to as a single variable domain fragment or nanobody. Thus, the VHH domain differs from the conventional VH domain of a four-chain immunoglobulin in that it does not need to be paired with a light chain variable domain to form an antigen-binding site. Such a VHH domain molecule may be derived from antibodies produced in Camelidae species such as camels, alpacas, dromedaries, llamas, and guanacos. Species other than Camelidae may also produce heavy chain antibodies naturally devoid of light chains, and such VHHs are also within the scope of the present invention. In some cases, for therapeutic applications of VHH domains, it is desirable to reduce their immunogenicity. Thus, preferably, in one embodiment, the VHH domain used in the present invention is a humanized VHH domain or a further sequence-optimized form thereof (e.g., an affinity-matured form to increase the binding affinity).

"Single-domain antibody" or "sdAb" is used herein to refer to an antibody polypeptide that recognizes and binds to an antigen of interest via a single variable antibody domain, e.g., a VHH or a single VH or a single VL. The single variable antibody domain of the single-domain antibody is capable of recognizing and binding to an antigen of interest without being paired with another antibody variable domain. Herein, the single-domain antibody comprising a heavy chain variable domain (VHH) of a heavy chain antibody is also referred to as a VHH single-domain antibody. The VHH single-domain antibody used in the present invention is preferably derived from animals of the Camelidae family, such as an alpaca, or a humanized form or a sequence-optimized form thereof. In some embodiments, the VHH single-domain antibody of the present invention is a monovalent monospecific polypeptide molecule consisting of, or consisting substantially of, a single VHH domain.

The single-domain antibody or VHH domain of the present invention may also be comprised in a larger polypeptide/protein. Examples of the polypeptide/protein comprising the single-domain antibody of the present invention that may be mentioned include, but are not limited to, heavy chain antibodies (HcAbs), e.g., heavy chain antibodies having framework regions and/or heavy chain constant regions derived from animals of the Camelidae family (llamas, camels, particularly alpacas), humanized forms thereof or sequence-optimized forms thereof (affinity-matured forms), or fragments thereof (e.g., fragments comprising at least part of a constant region); fusion proteins formed by a VHH domain from the heavy chain antibody as a component, e.g., with (part of) an immunoglobulin constant region (e.g., an Fc region). In one embodiment, the VHH domain of the present invention is fused to an Fc region, such as a human IgG1 Fc region, to form a VHH-Fc antibody.

Herein, the term "flexible linker peptide" or "linker peptide" or "linker" refers to a short amino acid sequence consisting of amino acids, such as glycine (G) and/or serine (S) and/or threonine residues (T) used alone or in combination, or a hinge region derived from an immunoglobulin. In one embodiment, the linker peptide has a length of 5-50 amino acids, such as 10, 15, 20, 25, or 30 amino acids. In one embodiment, the linker peptide comprises amino acid sequence (G4S)n, wherein n is an integer equal to or greater than 1, and, for example, n is an integer of 2, 3, 4, 5, 6, or 7. In one embodiment, the linker peptide comprises the amino acid sequence TS(G4S)n, wherein n is an integer equal to or greater than 1, and, for example, n is an integer of 2, 3, 4, 5, 6, or 7. In one embodiment, the linker peptide comprises the amino acid sequence G(G4S)n, wherein n is an integer equal to or greater than 1, and, for example, n is an integer of 2, 3, 4, 5, 6, or 7. In yet another embodiment, the linker peptide is a hinge region derived from an immunoglobulin, e.g., a hinge region amino acid sequence comprising "CPPC", such as an amino acid sequence "EPKSCDKTHTCPPCP" or "EPKSSDKTHTCPPCP". The linker peptide that may be used to link domains of the antibody molecules of the present invention may also be, for example, but is not limited to, the following amino acid sequences: GGG; DGGGS; TGEKP; GGRR; EGKSSGSGSESKVD; KESGSVSSEQLAQFRSLD; GGRRGGGS; LRQRDGERP; LRQKDGGGSERP; and GSTSGSGKPGSGEGSTKG. Alternatively, a computer program can be used to simulate three-dimensional structures of proteins and peptides, or a suitable flexible linker peptide is rationally designed by a phage display method.

As used herein, the term "binding" or "specific binding" means that the binding effect is selective for antigens and may be distinguished from unwanted or non-specific interactions. The ability of an antigen-binding site to bind to a particular antigen can be determined by an enzyme-linked immunosorbent assay (ELISA) or a conventional binding assay known in the art.

"Affinity" or "binding affinity" refers to the inherent binding affinity that reflects the interaction between members of a binding pair. The affinity of molecule X for its partner Y can be generally represented by a dissociation constant (KD), which is a ratio of the dissociation rate constant to the association rate constant (kdis and kon, respectively). Affinity can be measured by common methods known in the art. One specific method for measuring affinity is the ForteBio kinetic binding assay described herein.

The "percent identity (%)" of an amino acid sequence refers to the percentage of amino acid residues in a candidate sequence that are the same as those of a specific amino acid sequence shown in this specification when aligning the candidate sequence with the specific amino acid sequence shown in this specification, with gaps introduced if necessary to achieve maximum percent sequence identity and without considering any conservative replacements as part of sequence identity. In some embodiments, the present invention considers variants of the antibody molecule of the present invention that have a considerable degree of identity to the antibody molecule and sequence thereof specifically disclosed herein, and, for example, the identity is at least 80%, 85%, 90%, 95%, 97%, 98%, 99% or higher. The variants may comprise conservative modifications.

For polypeptide sequences, "conservative modifications" include replacements of, deletions of, or additions to a polypeptide sequence that do not substantially alter the desired functional activity of the polypeptide sequence. For example, conservative substitutions often result in the replacement of an amino acid with a chemically similar amino acid. Conservative replacement tables providing functionally similar amino acids are well known in the art. 8 groups comprising amino acids that are conservatively substituted with each other are listed as follows: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M) and valine (V); 6) phenylalanine (F), tyrosine (Y), and tryptophan (W); 7) serine (S) and threonine (T); and 8) cysteine (C) and methionine (M). In some embodiments, the term "conservative sequence modification" is used to refer to an amino acid modification that does not significantly affect or alter the binding characteristics for an antigen of interest of the antibody molecule or binding protein molecule of the present invention comprising the amino acid sequence. For example, conservatively modified variants retain at least 80%, 85%, 90%, 95%, 98%, 99%, or more, such as 100%-110% or more, binding affinity for an antigen of interest relative to the parent antibody or binding protein.

The term "host cell" refers to a cell into which an exogenous polynucleotide has been introduced, including the progeny of such cells. Host cells include "transformants" and "transformed cells", which include primary transformed cells and progeny derived therefrom. Host cells are any type of cell system that can be used to produce the antibody molecule of the present invention, including eukaryotic cells, e.g., mammalian cells, insect cells, and yeast cells; and prokaryotic cells, e.g., *E*. *coli* cells. Host cells include cultured cells, as well as cells within a transgenic animal, a transgenic plant, or cultured plant tissue or animal tissue.

The term "expression vector" refers to a vector comprising a recombinant polynucleotide, which comprises an expression control sequence operably linked to a nucleotide sequence to be expressed. Expression vectors contain sufficient cis-regulatory elements for expression, and other elements for expression may be provided by a host cell or in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) incorporated into recombinant polynucleotides.

The terms "individual" and "subject" are used interchangeably and refer to a mammal. The mammals include, but are not limited to, domesticated animals (e.g., cows, sheep, cats, dogs, and horses), primates (e.g., human and non-human primates such as monkeys), rabbits and rodents (e.g., mice and rats). In particular, individuals are humans. The term "treatment" refers to a clinical intervention intending to alter the natural progress of a disease in an individual being treated. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis. In some embodiments, the antibody molecule of the present invention is used to delay the progression of a disease or to slow the progression of a disease.

The term "anti-tumor effect" or "tumor inhibitory effect" refers to a biological effect that may be demonstrated by a variety of means, including but not limited to, for example, decrease in tumor volume, decrease in the number of tumor cells, decrease in tumor cell proliferation, or decrease in tumor cell viability. The terms "tumor" and "cancer" are used interchangeably herein to encompass a variety of solid tumors.

Herein, the term "neovascularization-associated disease" refers to a disease, disorder, and/or condition in which the onset, development, and/or progression of the disease involves neovascularization (including neovascularization, neolymphangiogenesis, and/or both). Such diseases, disorders, or conditions would benefit from the blocking of the biological activity of VEGF-C or VEGF-A or both.

Aspects of the present invention are described in detail below.

### I. Single-domain antibody polypeptide and derivative thereof

A single-domain antibody or VHH domain has a molecular weight of about one tenth of that of a human IgG molecule, and a physical diameter of only a few nanometers. Due to the small molecular size, a single-domain monoclonal antibody has the following advantages over conventional four-chain antibodies: high stability and solubility, and the ability to recognize hidden antigenic sites. In addition, the single-domain antibody is also cheaper to be prepared than the conventional four-chain antibodies, and may be easily expressed at higher expression levels and purified in *E*. *coli.* In addition to the use thereof as an individual molecule, the single-domain antibody is also a suitable component for the construction of multispecific molecules.

After intensive studies, in one aspect, the present invention provides a single-domain antibody and a derivative thereof. The single-domain antibody of the present invention specifically binds to human VEGFC and preferably blocks its binding to a receptor and the signaling induced thereby. In some embodiments, the single-domain antibody of the present invention has a biological activity selected from any one or more of the following:
(i) binding to human VEGF-C (hVEGF-C) with high affinity, preferably with a monovalent binding affinity KD value of about 1-10 nM, such as about 5-6 nM, or a bivalent binding affinity KD value of about 0.1-1 nM, such as about 0.6 nM, as determined by bio-layer interferometry, such as the method described in Example 3;
(ii) blocking the binding of hVEGF-C to a receptor VEGFR2, preferably with an IC50 value of about 0.1-0.5 nM, more preferably about 0.01-0.05 nM, as determined by an ELISA assay, such as the method described in Example 4;
(iii) blocking the binding of hVEGF-C to a receptor VEGFR3, preferably with an IC50 value of about 0.1-0.5 nM, more preferably about 0.01-0.05 nM, as determined by the ELISA assay, such as the method described in Example 4;
(iv) inhibiting the activation of a signaling pathway mediated by the binding of VEGFC-C to VEGFR2, preferably, as determined by a receptor-reporter system, such as the method described in Example 5;
(v) inhibiting the activation of a signaling pathway mediated by the binding of VEGFC-C to VEGFR3;
(vi) blocking and inhibiting vascular endothelium-associated activities induced by the binding of VEGFC to VEGFR2, such as vascular endothelial cell survival, proliferation, and/or migration, neovascularization, and/or vascular leakage; and
(vii) blocking and inhibiting the survival and/or proliferation of vascular endothelial cells and/or lymphatic endothelial cells induced by the binding of VEGFC to VEGFR3.

Structurally, like other conventional antibodies, the sdAb of the present invention has a modular structure, wherein a VHH variable region sequence comprises three CDRs and highly conserved 4 framework regions, and generally has a structure of the following formula: FR1-CDR-FR2-CDR2-FR3-CDR3-FR4, wherein FR1 to FR4 refer to framework regions 1 to 4; CDR1 to CDR3 refer to complementarity determining regions 1-3. The CDR sequences in the VHH variable region may be determined according to any of the CDR definition schemes described in the "Definition" section; preferably, the boundaries of the three CDRs in the sdAb variable region sequence may be defined according to Kabat, Chothia, or a combination thereof.

In some embodiments, the present invention provides a single-domain antibody expressed and isolated in a prokaryotic host cell, such as *E*. *coli.* In still other embodiments, the present invention provides a single-domain antibody expressed and isolated in a eukaryotic host cell, such as a mammalian cell, e.g., a CHO cell or 293 cell.

In some embodiments, the single-domain antibody of the present invention comprises CDR amino acid sequences and/or framework region (FR) amino acid sequences derived from a Camelidae heavy chain antibody produced by immunizing animals of the Camelidae family (e.g., an alpaca). In some embodiments, the single-domain monoclonal antibody of the present invention derived from the Camelidae heavy chain antibody may be engineered, for example, to comprise framework region and/or constant region sequences derived from human amino acid sequences (i.e., human antibodies) or other non-Camelidae mammalian species. For example, a framework region, a portion or all of a heavy chain constant region (e.g., CH1, CH2, or CH3 regions alone, or any combination thereof, such as a CH2-CH3 region), an immunoglobulin Fc region or a fragment thereof, and/or a hinge region or a portion of a hinge region, may be comprised in the antibody. In one embodiment, to further improve the properties (e.g., affinity) of the engineered antibody, Camelidae amino acid residues at corresponding positions in the parent Camelidae antibody may be introduced in the engineered antibody by back mutations at one or more positions (e.g., framework regions).

In one embodiment, the single-domain antibody or VHH domain of the present invention is humanized. The humanization may be achieved by the following method: replacing one or more amino acid residues, particularly framework region sequences, of a native VHH sequence of a non-human origin (e.g., a VHH sequence derived from animals of the Camelidae family or the alpaca after immunization) with residues from the heavy chain VH of the conventional human antibody at the corresponding positions. Methods for humanizing the single-domain monoclonal antibody are well known in the art. Typically, the humanization replacements are made in a manner that preserves the favorable binding properties of the single-domain antibody. Assays for determining biological properties of the humanized single-domain antibody, such as binding affinity and the like, are well known in the art to determine and select a suitable humanized residue mutation or a combination of mutations.

In some embodiments, the humanized single-domain antibody of the present invention may be obtained by a method comprising the following steps:
① determining a CDR loop structure of a parent single-domain antibody (e.g., a Camelidae VHH antibody screened from a phage display library);
② searching a human germline sequence database for the closest homologous sequences for each V/J region;
③ screening human germlines for the highest match in a heavy chain and a minimum quantity of back mutations;
④ constructing the CDR regions of the chimeric antibody onto framework regions of a human antibody;
⑤ determining amino acid positions that maintain CDR functions in the framework regions based on the sequences and structural features;
⑥ adding back mutations (back to the input amino acids) at important sequence positions identified;
⑦ optimizing amino acids at risk sites; and
⑧ obtaining the humanized antibody, optionally sequencing the antibody VHH sequence.

In some embodiments, the present invention further provides a functional variant of the single-domain antibody of the present invention. The functional variant may be obtained by the methods well known to the present invention, for example, by introducing mutations into the encoding nucleic acid sequences of exemplary single-domain antibodies of the present invention, e.g., into CDR sequences and/or FR sequences, and then screening (e.g., by phage display library screening) variants that retain the desired properties, e.g., via random or site-directed mutagenesis. Typically, the functional variant retains significant sequence identity to the parent single-domain antibody (or VHH domain). Preferably, the functional variant retains the desired biological properties of the parent single-domain antibody (or VHH domain), for example, the variant has equivalent (e.g., at least 50%, 60%, 70%, or 80%, preferably 90% or more) or improved biological activity (e.g., 110%-150% or more) relative to the biological activity of the parent. The desired biological properties include, for example, but are not limited to, binding affinity for an antigen of interest (as measured by KD values), activity to block the binding of an antigen of interest to a receptor (as measured by IC50 values), inhibitory activity to block the activation of a signaling pathway induced by an antigen of interest (as measured by IC50 values), blocking neovascularization and/or vascular leakage in *in vitro* or *in vivo* assays, and inhibiting tumor growth/survival in *in vitro* or *in vivo* assays.

In some embodiments, the present invention provides an affinity variant of the single-domain antibody polypeptide of the present invention. Preferably, the affinity variant exhibits one or more amino acid changes in the amino acid sequence relative to the parent single-domain antibody from which it is derived, wherein the affinity variant has altered binding affinity for an antigen of interest as compared to the parent antibody. Typically, the affinity variant exhibits improved antigen-binding affinity over the parent. The improvement may be, but is not limited to, a lower KD value, a faster dissociation rate, or an increase (or decrease) in cross-reactivity with a homologous protein from a non-human species of an antigen of interest. The affinity variant often has one or more amino acid residue substitutions in the CDRs as compared to the parent. The substitution may be a conservative or non-conservative substitution. In one embodiment, the affinity variant of the present invention has no more than 10, no more than 6, or 1-5, such as 1, 2, 3, 4, or 5, amino acid substitutions in total in the CDR1-3 relative to the parent. In some embodiments, mutations are introduced into CDR2 of the affinity variant relative to the parent. In another embodiment, mutations are introduced into CDR2 and CDR3 of the affinity variant relative to the parent. The affinity variant may be obtained by a variety of affinity maturation methods known in the art, including mutating CDRs, using *E*. *coli* mutator strains, DNA shuffling, phage display, and the like.

"Complementarity determining region", "CDR region", or "CDR" (used interchangeably herein with a hypervariable region "HVR") is an amino acid region in a variable region of an antibody that is primarily responsible for binding to an epitope. Herein, the CDRs of the single-domain antibody or VHH domain are generally referred to as CDR1, CDR2, and CDR3, which are numbered sequentially from the N-terminus. In some embodiments, the present invention provides an anti-VEGFC single-domain antibody, wherein the antibody comprises a VHH domain having the following CDR sequences:
(i) three CDR sequences of a VHH sequence set forth in SEQ ID NO: 4,
(ii) three CDR sequences of a VHH sequence set forth in SEQ ID NO: 8,
(iii) three CDR sequences of a VHH sequence set forth in SEQ ID NO: 12,
(iv) three CDR sequences of a VHH sequence set forth in SEQ ID NO: 16,
(v) three CDR sequences of a VHH sequence set forth in SEQ ID NO: 20, or
(vi) three CDR sequences of a VHH sequence set forth in SEQ ID NO: 24;

preferably, the CDR sequences are defined according to kabat or defined according to Chothia; or CDR1 is defined according to a combination of Kabat and Chothia, and CDR2 and CDR3 are defined according to Kabat;
   or,
wherein the antibody comprises a variant of the CDR sequences selected from any one of (i)-(vi) described above, for example, the variant has 1-8, 1-5, or 1, 2, 3, 4, 5, or 6 amino acid changes, preferably conservative amino acid substitutions relative to the CDR sequences according to any one of (i)-(vi) described above.

In some embodiments, the present invention provides an anti-VEGFC single-domain antibody, which comprises a VHH domain of the following formula consisting of 3 CDRs and 4 FRs: FR1-CDR1-FR2-CDR2-FR3-CDR3-FR4; wherein
(i) the CDR1 comprises or consists of an amino acid sequence set forth in SEQ ID NO: 1, the CDR2 comprises or consists of an amino acid sequence set forth in SEQ ID NO: 2, and the CDR3 comprises or consists of an amino acid sequence set forth in SEQ ID NO: 3;
(ii) the CDR1 comprises or consists of an amino acid sequence set forth in SEQ ID NO: 9, the CDR2 comprises or consists of an amino acid sequence set forth in SEQ ID NO: 10, and the CDR3 comprises or consists of an amino acid sequence set forth in SEQ ID NO: 11;
(iii) the CDR1 comprises or consists of an amino acid sequence set forth in SEQ ID NO: 17, the CDR2 comprises or consists of an amino acid sequence set forth in SEQ ID NO: 18, and the CDR3 comprises or consists of an amino acid sequence set forth in SEQ ID NO: 19; or
(iv) the CDR1 comprises or consists of an amino acid sequence set forth in SEQ ID NO: 21, the CDR2 comprises or consists of an amino acid sequence set forth in SEQ ID NO: 22, and the CDR3 comprises or consists of an amino acid sequence set forth in SEQ ID NO: 23;
or
wherein the antibody comprises a variant of the CDR sequences selected from any one of (i)-(vi) described above, for example, the variant has 1-8, 1-5, or 1, 2, 3, 4, 5, or 6 amino acid changes, preferably conservative amino acid substitutions relative to the CDR sequences according to any one of (i)-(vi) described above.

Preferably, the anti-VEGFC single-domain antibody of the present invention comprises a VHH domain of the following formula: FR1-CDR1-FR2-CDR2-FR3-CDR3-FR4, wherein the CDR1 comprises or consists of the amino acid sequence set forth in SEQ ID NO: 17, the CDR2 comprises or consists of the amino acid sequence set forth in SEQ ID NO: 18, and the CDR3 comprises or consists of the amino acid sequence set forth in SEQ ID NO: 19.

More preferably, the anti-VEGFC single-domain antibody of the present invention comprises a VHH domain of the following formula: FR1-CDR1-FR2-CDR2-FR3-CDR3-FR4, wherein the CDR1 comprises or consists of the amino acid sequence set forth in SEQ ID NO: 21, the CDR2 comprises or consists of the amino acid sequence set forth in SEQ ID NO: 22, and the CDR3 comprises or consists of the amino acid sequence set forth in SEQ ID NO: 23.

The "variable region" or "variable domain" of a single-domain antibody is a region in the single-domain antibody that is involved in antigen binding. The heavy chain variable domain in the single-domain antibody is also referred to herein as a VHH domain. It is known in the art that one or more residues in the VHH variable region may be modified, for example, one or more CDR regions and/or one or more framework regions undergo residue modifications, particularly conservative residue substitutions, and the modified antibody still substantially retains at least one biological property (e.g., antigen-binding ability) of the antibody molecule prior to the modification.

For example, residues in CDR regions may be mutated to improve one or more binding properties (e.g., affinity) of the antibody. The antigen-binding properties or other functional properties of the mutated antibody can be assessed in an *in vitro* or *in vivo* assay. Preferably, conservative substitutions are introduced. Preferably, no more than 1, 2, 3, 4, or 5 residue modifications are introduced in the CDR regions. In addition, residues in framework regions can be mutated, for example, to improve the properties of the antibody. For example, one or more residues in the framework regions may be "back mutated" to corresponding residues of a germline sequence.

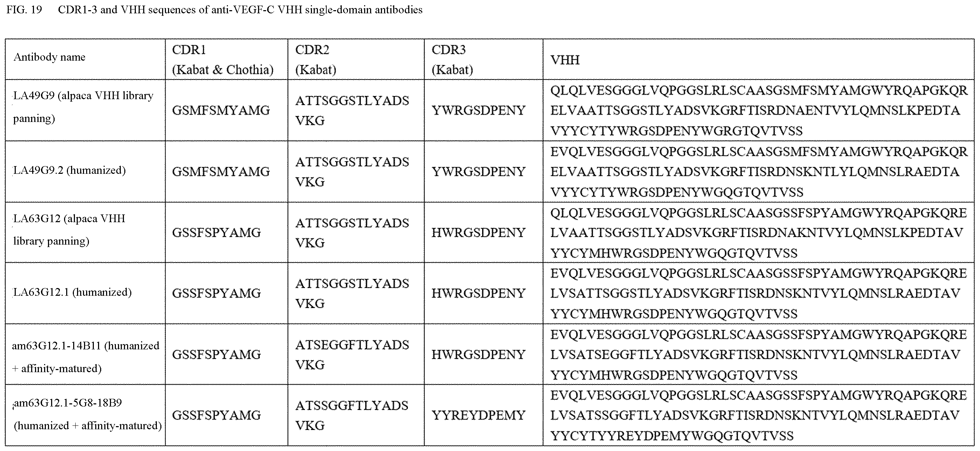

CDR grafting is another modification method for antibody variable region known in the art. Since CDR sequences are responsible for most of the antibody-antigen interactions, a recombinant antibody variant that simulates the properties of known antibodies can be constructed. In the antibody variant, CDR sequences from the known antibodies are grafted onto framework regions of different antibodies having different properties. Thus, in one embodiment, the present invention relates to an anti-VEGFC single-domain antibody, which comprises CDR sequences of the heavy chain variable region from one of the exemplary VHH single-domain antibodies in FIG. 19, but has different framework region sequences. A framework region sequence for substitution may be obtained from a public DNA database, including germline antibody gene sequences, or from published VEGFC antibody sequences. For example, germline DNAs encoding human heavy chain variable region genes may be obtained from the GenBank database. Antibody protein sequences can be compared to protein sequences in the database using sequence similarity search tools, such as Gapped BLAST. Preferably, the framework sequence for substitution is structurally similar to a framework sequence of the antibody of the present invention selected for change, e.g., a framework sequence having at least 80%, 85%, 90%, 95%, 96%, 97%, 98%, 99% or more sequence identity. In some embodiments, the humanization of the antibody may be performed according to the method of Example 7.

Thus, in one embodiment, the present invention provides an anti-VEGFC single-domain antibody, which comprises or consists of an amino acid sequence selected from SEQ ID NOs: 4, 8, 12, 16, 20, and 24, or an amino acid sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more identity thereto.

In another embodiment, the present invention provides an anti-VEGFC single-domain antibody, wherein the antibody:
(i) comprises a VHH sequence having the amino acid sequence set forth in SEQ ID NO: 4 or a variant thereof; or
(ii) comprises a VHH sequence having the amino acid sequence set forth in SEQ ID NO: 8 or a variant thereof; or
(iii) comprises a VHH sequence having the amino acid sequence set forth in SEQ ID NO: 12 or a variant thereof; or
(iv) comprises a VHH sequence having the amino acid sequence set forth in SEQ ID NO: 16 or a variant thereof; or
(v) comprises a VHH sequence having the amino acid sequence set forth in SEQ ID NO: 20 or a variant thereof; or
(vi) comprises a VHH sequence having the amino acid sequence set forth in SEQ ID NO: 24 or a variant thereof,
wherein with respect to the amino acid sequence, the variant has at least 80%, 85%, 90%, 92%, 95%, 97%, 98%, 99%, or more identity relative to the reference VHH sequence (preferably, in terms of the full-length, or the three regions of CDR1, CDR2, and CDR3). In one embodiment, with respect to the amino acid sequence, the variant comprises at least one and no more than 30, 10, 5, 4, 3, 2, 1, or 0 amino acid changes (preferably amino acid substitutions, and more preferably conservative substitutions) relative to the reference VHH sequence (preferably, in terms of the full-length, or the three regions of CDR1, CDR2, and CDR3). Preferably, the difference between the variant and the reference VHH sequence is not in the CDR regions.

Preferably, the anti-VEGFC single-domain antibody of the present invention comprises or consists of the amino acid sequence set forth in SEQ ID NO: 20. More preferably, the anti-VEGFC single-domain antibody of the present invention comprises or consists of the amino acid sequence set forth in SEQ ID NO: 24.

### II. Fusion protein/chimeric polypeptide

In a further aspect, the present invention provides a polypeptide comprising at least one single-domain antibody or VHH domain of the present invention, wherein the polypeptide comprises the single-domain monoclonal antibody or VHH domain of the present invention and other peptide/polypeptide sequences linked at its N-terminus or C-terminus or both N- and C-termini.

In some embodiments, the single-domain antibody or VHH domain of the present invention (e.g., a Camelidae VHH domain or a humanized form thereof) may be linked to a constant region (e.g., an Fc region) of a human antibody to produce a VHH-Fc polypeptide. In one embodiment, the VHH-Fc polypeptide comprises the single-domain antibody of the present invention and an Fc portion at its C-terminus.

In yet another embodiment, the VHH-Fc polypeptide comprises an Fc portion derived from animals of the Camelidae family. In one embodiment, the VHH-Fc polypeptide is produced and isolated by immunizing animals of the Camelidae family such as an alpaca. A variety of methods known in the art may be used for immunizing animals of the Camelidae family and isolating the VHH antibody produced against the antigen of interest.

In still other embodiments, the VHH-Fc polypeptide comprises an Fc portion from a human or non-human primate. In yet another embodiment, the VHH-Fc polypeptide comprises a human IgG Fc region, e.g., a human IgG1 Fc region, such as an Fc region comprising an amino acid sequence set forth in SEQ ID NO: 27 or an amino acid sequence having at least 85%, 86%, 87%, 88%, 89%, 90%, 91%, 92%, 93%, 94%, 95%, 96%, 97%, 98%, 99%, or more identity thereto.

In one embodiment, the VHH-Fc polypeptide according to the present invention comprises an Fc portion fused to the C-terminus of the single-domain monoclonal antibody via a linker peptide, such as a hinge region.

In yet another embodiment, the VHH-Fc polypeptide according to the present invention may be dimerized with another polypeptide chain comprising an Fc portion (e.g., another VHH-Fc polypeptide, which is the same or different) via the Fc portion. Thus, in yet another embodiment, the present invention further provides a homo- or heteromultimeric protein comprising the VHH-Fc polypeptide of the present invention. In a preferred embodiment, the protein preferably comprises a heavy chain antibody formed by pairing two identical VHH-Fc polypeptide chains.

In addition to the VHH-Fc polypeptide and heavy chain antibody described above, the present invention further provides other fusion proteins/chimeric polypeptides comprising at least one single-domain antibody polypeptide according to the present invention.

In some embodiments, the fusion protein/chimeric polypeptide of the present invention comprises a single single-domain antibody or VHH domain according to the present invention. In another embodiment, the fusion protein/chimeric polypeptide of the present invention comprises a plurality of single-domain antibodies or VHH domains according to the present invention. The fusion protein according to the present invention may be monospecific, or may be multispecific. In some embodiments, the fusion protein/chimeric polypeptide of the present invention comprises specificity for VEGFC only. In yet another embodiment, the fusion protein/chimeric polypeptide of the present invention, in addition to comprising the single-domain antibody or VHH domain(s) of the present invention directed against VEGFC, comprises specificity for another antigen, such as VEGFA.

As understood by those skilled in the art, each component in the fusion protein is operably linked to allow it to perform its intended function. In one embodiment, the components of the fusion protein may be linked directly to each other, or linked via a linker peptide or linker. In some embodiments, the linker comprises an amino acid sequence from an immunoglobulin hinge region, or comprises a flexible amino acid sequence, e.g., a sequence consisting of glycine and serine, such as (G4S)n or G(G4S)n, wherein n = 1, 2, 3, 4, 5, 6, or 7, preferably 2, 3, or 4.

### III. Bispecific binding molecule

In a further aspect, the present invention provides an anti-VEGFC/VEGFA bispecific binding molecule, which comprises:
(i) a first antigen-binding component specifically binding to VEGF-C; and
(ii) a second antigen-binding component specifically binding to VEGF-A.

The bispecific molecule of the present invention provides dual antagonistic activity against VEGF-A and VEGF-C.

In some embodiments, the bispecific binding molecule of the present invention has a biological activity selected from any one or more of the following:
(i) blocking the activation of a VEGFR2 signaling pathway induced by hVEGF-A alone, preferably, as determined by measuring an IC50 value for the inhibitory activity using a KDR reporter assay, such as the method described in Example 10, wherein the IC50 value is, for example, 0.1-10 nM, e.g., about 0.2-1 nM, such as 0.6 nM, or about 1-3 nM, such as about 1 nM;
(ii) blocking the activation of a VEGFR2 signaling pathway induced by hVEGF-C alone, preferably, as determined by measuring an IC50 value for the inhibitory activity using the KDR reporter assay, such as the method described in Example 10, wherein the IC50 value is 0.1-1 nM, such as about 0.2-0.6 nM;
(iii) inhibiting the proliferation of lymphatic vessel cells induced by hVEGF-C, preferably, as determined by measuring an IC50 value for the inhibitory activity using a cell proliferation assay, such as the method described in Example 11, wherein the IC50 value is 0.01-0.5 nM, such as about 0.1-0.5 nM;
(iv) inhibiting the survival and proliferation of endothelial cells induced by both hVEGF-C and hVEGF-A at an inhibitory level of at least 80%, 85%, 90%, 95%, or about 100%, preferably, as determined by measuring an IC50 value for the inhibitory activity using a cell proliferation assay, such as the method described in Example 12, wherein the IC50 value is 0.1-0.5 nM, such as about 0.2 nM;
(v) inhibiting the formation of neovascular structures induced by both hVEGF-C and hVEGF-A at an inhibitory level of at least 80%, 85%, 90%, 95%, or about 100%, preferably, as determined by measuring the inhibitory activity using an endothelial cell tube formation assay, such as the method described in Example 13;
(vi) inhibiting neovascularization of a tumor (e.g., a solid tumor, such as melanoma);
(vii) inhibiting the growth of a tumor (e.g., a solid tumor, such as melanoma), e.g., with a tumor growth inhibition of 50% or more after single drug administration, for example, in a tumor-bearing animal model, such as the tumor-bearing animal model described in Example 14; and
(viii) blocking and inhibiting the development and/or progression of neovascularization-associated ocular diseases, for example, reducing the level of neovascularization, reducing vascular leakage, or inhibiting retinal edema and/or thickening of the fundus caused by neovascularization.

Thus, in some embodiments, the present invention provides an anti-VEGFA/VEGFC bispecific binding molecule that may be used for the treatment of neovascularization-associated diseases, such as cancers (e.g., solid tumors) and ocular diseases (e.g., age-related macular degeneration (AMD)).

The anti-VEGFC component of the anti-VEGFA/VEGFC bispecific binding molecule according to the present invention may comprise any of the anti-VEGFC single-domain antibody polypeptides of the present invention or any of the anti-VEGFC VHH domains according to the present invention described above. Preferably, the anti-VEGFC component comprises or consists of the anti-VEGFC VHH domain according to the present invention, and more preferably, the VHH domain is a humanized VHH domain. In one embodiment, the VHH domain comprises CDR1, CDR2, and CDR3 amino acid sequences set forth in SEQ ID NOs: 1, 2, and 3, respectively. In one embodiment, the VHH domain comprises CDR1, CDR2, and CDR3 amino acid sequences set forth in SEQ ID NOs: 9, 10, and 11. In a preferred embodiment, the VHH domain comprises CDR1, CDR2, and CDR3 amino acid sequences set forth in SEQ ID NOs: 17, 18, and 19. In yet another preferred embodiment, the VHH domain comprises CDR1, CDR2, and CDR3 amino acid sequences set forth in SEQ ID NOs: 21, 22, and 23. In yet another embodiment, the VHH domain comprises (i) an amino acid sequence selected from SEQ ID NO: 4, 8, 12, 16, 20, or 24, or (ii) an amino acid sequence having at least 80%, 85%, 90%, 91%, 92%, 93%, 94%, 95%, 96%, 97%, 98%, 99%, or more identity to the amino acid sequence of (i), or (iii) an amino acid sequence having at least 1-30, 1-20, 1-15, 1-10, or 1-5 amino acid changes (e.g., substitutions, deletions, and/or insertions, preferably substitutions, and more preferably conservative substitutions) relative to the amino acid sequence of (i). In a preferred embodiment, the VHH domain comprises or consists of an amino acid sequence set forth in SEQ ID NO: 20 or 24. In one embodiment, the anti-VEGFA/VEGFC bispecific binding molecule according to the present invention comprises at least one, such as two or more (preferably two) anti-VEGFC single-domain antibodies or VHH domains of the present invention.

The anti-VEGFA component of the anti-VEGFA/VEGFC bispecific binding molecule according to the present invention may be provided by any molecule comprising a VEGFA binding domain, for example, but not limited to, anti-VEGFA antibodies, such as single-chain Fv antibodies, Fab antibodies, Fab' antibodies, (Fab)₂ antibodies, single-domain antibodies, and nanobodies; anti-VEGFA Trap molecules, such as Trap molecules comprising an extracellular domain of a VEGFA receptor VEGFR1, Trap molecules comprising an extracellular domain of a VEGFA receptor VEGFR2, or Trap molecules comprising extracellular domains of VEGFR1 and VEGFR2; and fusion proteins or chimeric polypeptides of the VEGFA binding domain and the Fc portion of an immunoglobulin. In the bispecific antibody molecule according to the present invention, the anti-VEGFC component is linked to or fused at the C-terminus or N-terminus of the anti-VEGFA component. In some embodiments, the anti-VEGFC component may be covalently linked to the N-terminus or C-terminus, preferably C-terminus, of the anti-VEGFA component either directly or, preferably, via a linker sequence. In some embodiments, the anti-VEGFA component according to the present invention binds to human VEGFA and inhibits the binding of VEGFA to its receptors VEGFR1 and/or VEGFR2 and signaling induced thereby.

In some preferred embodiments, the anti-VEGFA component according to the present invention is provided by an Fc chimeric polypeptide comprising a VEGFA binding domain, such that the anti-VEGFA component comprises a VEGFA binding domain in the form of an Fc fusion protein. In other preferred embodiments, the anti-VEGFA component is provided by an anti-VEGFA Fab antibody, such that the anti-VEGFA component comprises a VEGFA binding domain in the form of a Fab antibody. In embodiments where the anti-VEGFA component is an Fc fusion protein, the VEGFA binding domain may be preferably located at the N-terminus or C-terminus of the Fc portion, and the anti-VEGFC component is linked to the opposite terminus of the Fc portion. In embodiments where the anti-VEGFA component is a Fab antibody, the anti-VEGFC component is preferably linked to the C-terminus of the Fab antibody.

In some embodiments, the bispecific binding protein according to the present invention may comprise a linker connecting the anti-VEGFA component and the anti-VEGFC component. In one embodiment, the linker is a peptide of about 6 to about 30, such as 10, 11, 12, 13, 14, 15, 16, 17, 18, 19, 20, 21, 22, 23, 24, 25, 26, 27, 28, 29, or 30, amino acids in length. Preferably, the linker comprises an amino acid sequence (G4S)n or G(G4S)n, wherein n is an integer of 1, 2, 3, 4, or 5, preferably n = 2, 3, or 4.

### Bispecific binding molecule in the form of Fc fusion protein

Thus, in some embodiments, the present invention provides an anti-VEGFA/VEGFC bispecific binding molecule, wherein the binding molecule comprises an anti-VEGFA component and an anti-VEGFC component in the form of an Fc fusion protein, wherein the anti-VEGFA component comprises a VEGF-A binding domain fused to an immunoglobulin Fc region, and the anti-VEGF-C component comprises any anti-VEGF-C single-domain antibody polypeptide described in the present invention, particularly a VHH single-domain antibody polypeptide consisting of any anti-VEGF-C VHH domain described in the present invention. In one embodiment, the anti-VEGFC component is linked, preferably covalently linked, and more preferably linked via a linker to the Fc region of the anti-VEGFA component. In yet another embodiment, the VEGF-A binding domain and the anti-VEGF-C VHH domain are located at both termini of the Fc region, respectively. In some preferred embodiments, the VEGF-A binding domain is located at the N-terminus of the Fc region, and the anti-VEGF-C VHH domain is located at the C-terminus of the Fc region.

In one embodiment, the VEGF-A binding domain is a VEGF-A binding polypeptide. In yet another embodiment, the VEGF-A binding polypeptide comprises an extracellular VEGFA binding domain from a VEGFA receptor, such as VEGFR1 and/or VEGFR2. In one embodiment, the VEGFA binding polypeptide comprises a third Ig domain of VEGFR2 genetically fused to a second Ig domain of VEGFR1. In one embodiment, the VEGF-A binding polypeptide comprises or consists of an amino acid sequence set forth in SEQ ID NO: 26, or an amino acid sequence having at least 90%, 91%, 92%, 93%, 94%, 95%, 96%, 97%, 98%, 99%, or more identity thereto. Preferably, the VEGF-A binding polypeptide comprises or consists of the amino acid sequence set forth in SEQ ID NO: 26.

In one embodiment, the Fc region is an Fc region of a human IgG, such as a human IgG1 Fc region, preferably comprising or consisting of the amino acid sequence set forth in SEQ ID NO: 27, or an amino acid sequence having at least 90%, 91%, 92%, 93%, 94%, 95%, 96%, 97%, 98%, 99%, or more identity thereto.

In one embodiment, the anti-VEGFA component comprising a VEGFA binding polypeptide and an Fc region comprises or consists of the amino acid sequence set forth in SEQ ID NO: 25, or an amino acid sequence having at least 90%, 91%, 92%, 93%, 94%, 95%, 96%, 97%, 98%, 99%, or more identity thereto.

In some embodiments, the anti-VEGFA/VEGFC bispecific binding molecule of the present invention forms a dimer through the dimerization of the Fc regions.

In a preferred embodiment, in the anti-VEGFA/VEGFC bispecific binding molecule of the present invention, the anti-VEGFA binding polypeptide according to the present invention and the anti-VEGFC VHH domain according to the present invention are fused to the N-terminus and the C-terminus of the immunoglobulin Fc region, respectively, and the fusion polypeptide chain formed thereby forms a dimer through the dimerization of the Fc regions. Thus, in a preferred embodiment, the present invention provides an anti-VEGFA/VEGFC bispecific binding molecule comprising a first polypeptide chain and a second polypeptide chain, wherein the first polypeptide chain and the second polypeptide chain each comprise a fusion polypeptide formed by the fusion of the anti-VEGFA binding polypeptide according to the present invention and the anti-VEGFC VHH domain according to the present invention to the N-terminus and the C-terminus of the immunoglobulin Fc region, respectively, wherein the first polypeptide chain and the second polypeptide chain may be identical or different. Preferably, the first polypeptide chain and the second polypeptide chain are identical, and the anti-VEGFA/VEGFC bispecific binding molecule of the present invention is a homodimeric protein comprising the first polypeptide chain and the second polypeptide chain. In one embodiment, the first polypeptide chain and the second polypeptide chain are identical and each comprises a VEGF-A binding polypeptide consisting of the amino acid sequence set forth in SEQ ID NO: 26, or an amino acid sequence having at least 90%, 91%, 92%, 93%, 94%, 95%, 96%, 97%, 98%, 99%, or more identity thereto. In yet another embodiment, the first polypeptide chain and the second polypeptide chain are identical and each comprises an anti-VEGF-C VHH domain comprising CDR1, CDR2, and CDR3 sequences selected from amino acid sequences set forth in SEQ ID NO: 4, 8, 12, 16, 20, or 24; preferably, the VHH domain comprises CDR1, CDR2, and CDR3 sequences set forth in SEQ ID NOs: 1-3, SEQ ID NOs: 9-11, SEQ ID NOs: 17-19, or SEQ ID NOs: 21-23; more preferably, the VHH domain comprises or consists of an amino acid sequence selected from SEQ ID NO: 4, 8, 12, 16, 20, or 24, or an amino acid sequence having at least 90%, 91%, 92%, 93%, 94%, 95%, 96%, 97%, 98%, 99%, or more identity thereto. In yet another embodiment, the first polypeptide chain and the second polypeptide chain are identical and each comprises an Fc region from human IgG1. In yet another embodiment, the first polypeptide chain and the second polypeptide chain are identical, and the anti-VEGFC VHH domain according to the present invention is fused to the C-terminus of the immunoglobulin Fc region via a linker. In one embodiment, the linker comprises an amino acid sequence G(G4S)n or G(G4S)n, wherein n is an integer of 1, 2, 3, 4, or 5, preferably n = 2, 3, or 4. In yet another embodiment, the first polypeptide chain and the second polypeptide chain are identical and each comprises an amino acid sequence selected from SEQ ID NOs: 40-41 and 44-47, or an amino acid sequence having at least 85%, 90%, 95%, 96%, 97%, 98%, or 99% sequence identity thereto. Preferably, the first polypeptide chain and the second polypeptide chain are identical and each comprises an amino acid sequence set forth in SEQ ID NO: 47.

### Bispecific binding molecule in the form of Fab antibody

In still other embodiments, the present invention provides an anti-VEGFA/VEGFC bispecific binding molecule, wherein the binding molecule comprises an anti-VEGFA component and an anti-VEGFC component in the form of Fab antibodies, wherein the anti-VEGFA component comprises a heavy chain variable region VH and a light chain variable region VL that are paired to form a VEGF-A binding domain; the VH and the VL are fused to a heavy chain constant region CH1 and a light chain constant region CL of an immunoglobulin, respectively, to form an anti-VEGFA Fab antibody. In still other embodiments, the anti-VEGFC component is linked, preferably covalently linked, and more preferably linked via a linker, to the anti-VEGFA component in the form of the Fab antibody through the C-terminus of the CH1 and/or CL constant region of the anti-VEGFA Fab antibody. In yet another embodiment, the anti-VEGFA Fab antibody is linked to one anti-VEGF-C component at each of the C-termini of the CH1 constant region and the CL constant region, respectively. Preferably, the anti-VEGF-C component comprises any anti-VEGF-C single-domain antibody polypeptide described in the present invention, particularly a VHH single-domain antibody polypeptide consisting of any anti-VEGF-C VHH domain described in the present invention.

In some embodiments of the bispecific binding molecule according to the present invention, the anti-VEGFA Fab antibody according to the present invention comprises: a Fab heavy chain formed by linking the heavy chain variable region VH to the immunoglobulin heavy chain constant region CH1, and a Fab light chain formed by linking the light chain variable region VL to the immunoglobulin constant region VL. In another embodiment, the anti-VEGFA Fab antibody according to the present invention comprises: a Fab light chain formed by linking the heavy chain variable region VH to the immunoglobulin constant region VL, and a Fab heavy chain formed by linking the light chain variable region VL to the immunoglobulin heavy chain constant region CH1.

In some embodiments, the anti-VEGFA Fab antibody according to the present invention comprises a heavy chain variable region VH and a light chain variable region VL, wherein the VH comprises amino acid sequences of HCDR1-3 set forth in SEQ ID NOs: 34-36, and the VL comprises amino acid sequences of LCDR1-3 set forth in SEQ ID NOs: 37-39. In yet another embodiment, the anti-VEGFA Fab antibody is a chimeric antibody or a humanized antibody, preferably a humanized antibody. In yet another embodiment, the VEGF Fab antibody comprises a heavy chain variable region VH and a light chain variable region VL, wherein the VH comprises an amino acid sequence set forth in SEQ ID NO: 29, or an amino acid sequence having at least 80%, 85%, 90%, 91%, 92%, 93%, 94%, 95%, 96%, 97%, 98%, 99%, or more identity thereto, and the VL comprises an amino acid sequence set forth in SEQ ID NO: 32, or an amino acid sequence having at least 80%, 85%, 90%, 91%, 92%, 93%, 94%, 95%, 96%, 97%, 98%, 99%, or more identity thereto; preferably, the VH comprises the amino acid sequence set forth in SEQ ID NO: 29, and the VL comprises the amino acid sequence set forth in SEQ ID NO: 32.

In still other embodiments, the anti-VEGFA Fab antibody according to the present invention comprises a CH1 constant region of human immunoglobulin IgG1. In another embodiment, the anti-VEGFA Fab antibody according to the present invention comprises a human κ light chain constant region CL. In one embodiment, the anti-VEGFA Fab antibody comprises a heavy chain CH1 constant region having an amino acid sequence set forth in SEQ ID NO: 30, or an amino acid sequence having at least 80%, 85%, 90%, 91%, 92%, 93%, 94%, 95%, 96%, 97%, 98%, 99%, or more identity thereto. In yet another embodiment, the anti-VEGFA Fab antibody comprises a light chain CL constant region having an amino acid sequence set forth in SEQ ID NO: 33, or an amino acid sequence having at least 80%, 85%, 90%, 91%, 92%, 93%, 94%, 95%, 96%, 97%, 98%, 99%, or more identity thereto.

In some embodiments, the anti-VEGFA Fab antibody according to the present invention preferably comprises a Fab heavy chain consisting of the VH and the CH1 according to the present invention (VH-CH1 chain) and a Fab light chain consisting of the VL and the CL according to the present invention (VL-CL chain). More preferably, the anti-VEGFA Fab antibody according to the present invention comprises a Fab heavy chain having an amino acid sequence set forth in SEQ ID NO: 28, or an amino acid sequence having at least 80%, 85%, 90%, 91%, 92%, 93%, 94%, 95%, 96%, 97%, 98%, 99%, or more identity thereto; and a Fab light chain having an amino acid sequence set forth in SEQ ID NO: 31, or an amino acid sequence having at least 80%, 85%, 90%, 91%, 92%, 93%, 94%, 95%, 96%, 97%, 98%, 99%, or more identity thereto. More preferably, the anti-VEGFA Fab antibody according to the present invention comprises the amino acid sequence set forth in SEQ ID NO: 28 and the amino acid sequence set forth in SEQ ID NO: 31.

In still other embodiments of the bispecific binding molecule according to the present invention, the anti-Fab antibody according to the present invention is linked at the C-termini of the CH1 and the CL, directly or preferably via a linker, to the anti-VEGFC single-domain antibody of the present invention or to the anti-VEGFC VHH domain of the present invention, respectively. Thus, in one embodiment, the present invention provides an anti-VEGFA/VEGFC bispecific binding molecule comprising a first polypeptide chain and a second polypeptide chain, wherein the first polypeptide chain comprises the Fab heavy chain of the anti-VEGFA Fab antibody according to the present invention and the anti-VEGFC VHH domain linked to the C-terminus of the CH1 of the Fab heavy chain; the second polypeptide chain comprises the Fab light chain of the anti-VEGFA Fab antibody according to the present invention and the anti-VEGFC VHH domain linked to the C-terminus of the CL of the Fab light chain. Preferably, the Fab heavy chain consists of the VH and the CH1 (VH-CH1); the Fab light chain consists of the VL and the CL (VL-CL).

In a preferred embodiment, the present invention provides an anti-VEGFA/VEGFC bispecific binding molecule comprising a first polypeptide chain and a second polypeptide chain, wherein the first polypeptide chain comprises, from N-terminus to C-terminus, VH-CH1-linker-VHH, and the second polypeptide chain comprises, from N-terminus to C-terminus, VL-CL-linker-VHH, wherein the VHH is the anti-VEGFA VHH domain of the present invention; the VH-CH1 and VL-CL are paired to form the anti-VEGF-A Fab antibody of the present invention, wherein optionally, the CH1 and CL may also be linked directly to the VHH domain without a linker. In some embodiments, the VHH domain comprises CDR1, CDR2, and CDR3 sequences selected from amino acid sequences set forth in SEQ ID NO: 4, 8, 12, 16, 20, or 24; preferably, the VHH domain comprises CDR1, CDR2, and CDR3 sequences set forth in SEQ ID NOs: 1-3, SEQ ID NOs: 9-11, SEQ ID NOs: 17-19, or SEQ ID NOs: 21-23; more preferably, the VHH domain comprises or consists of an amino acid sequence selected from SEQ ID NO: 4, 8, 12, 16, 20, or 24, or an amino acid sequence having at least 90%, 91%, 92%, 93%, 94%, 95%, 96%, 97%, 98%, 99%, or more identity thereto; still more preferably, the VHH domain comprises an amino acid sequence set forth in SEQ ID NO: 20 or 24. In some embodiments, the VH comprises amino acid sequences of HCDR1-3 set forth in SEQ ID NOs: 34-36, and the VL comprises amino acid sequences of LCDR1-3 set forth in SEQ ID NOs: 37-39; preferably the VH and VL are humanized; more preferably, the VH comprises the amino acid sequence set forth in SEQ ID NO: 29, or an amino acid sequence having at least 80%, 85%, 90%, 91%, 92%, 93%, 94%, 95%, 96%, 97%, 98%, 99%, or more identity thereto, and the VL comprises the amino acid sequence set forth in SEQ ID NO: 32, or an amino acid sequence having at least 80%, 85%, 90%, 91%, 92%, 93%, 94%, 95%, 96%, 97%, 98%, 99%, or more identity thereto; still more preferably, the VH comprises the amino acid sequence set forth in SEQ ID NO: 29, and the VL comprises the amino acid sequence set forth in SEQ ID NO: 32. In some embodiments, the CH1 comprises the CH1 constant region of human immunoglobulin IgG1. In another embodiment, the CL comprises the human κ light chain constant region CL. In one embodiment, the CH1 comprises the amino acid sequence set forth in SEQ ID NO: 30, or an amino acid sequence having at least 80%, 85%, 90%, 91%, 92%, 93%, 94%, 95%, 96%, 97%, 98%, 99%, or more identity thereto. In yet another embodiment, the CL comprises the amino acid sequence set forth in SEQ ID NO: 33, or an amino acid sequence having at least 80%, 85%, 90%, 91%, 92%, 93%, 94%, 95%, 96%, 97%, 98%, 99%, or more identity thereto. In one embodiment, the linker comprises an amino acid sequence G(G4S)n or G(G4S)n, wherein n is an integer of 1, 2, 3, 4, or 5, preferably n = 2, 3, or 4.

In a further preferred embodiment, the present invention provides an anti-VEGFA/VEGFC bispecific binding molecule comprising a first polypeptide chain and a second polypeptide chain, wherein the first polypeptide chain and the second polypeptide chain are selected from:
- a first polypeptide chain comprising an amino acid sequence set forth in SEQ ID NO: 42, or an amino acid sequence having at least 85%, 90%, 95%, 96%, 97%, 98%, or 99% sequence identity thereto, and a second polypeptide chain comprising an amino acid sequence set forth in SEQ ID NO: 43, or an amino acid sequence having at least 85%, 90%, 95%, 96%, 97%, 98%, or 99% sequence identity thereto;
- a first polypeptide chain comprising an amino acid sequence set forth in SEQ ID NO: 48, or an amino acid sequence having at least 85%, 90%, 95%, 96%, 97%, 98%, or 99% sequence identity thereto, and a second polypeptide chain comprising an amino acid sequence set forth in SEQ ID NO: 49, or an amino acid sequence having at least 85%, 90%, 95%, 96%, 97%, 98%, or 99% sequence identity thereto; and
- a first polypeptide chain comprising an amino acid sequence set forth in SEQ ID NO: 50, or an amino acid sequence having at least 85%, 90%, 95%, 96%, 97%, 98%, or 99% sequence identity thereto, and a second polypeptide chain comprising an amino acid sequence set forth in SEQ ID NO: 51, or an amino acid sequence having at least 85%, 90%, 95%, 96%, 97%, 98%, or 99% sequence identity thereto.

In a preferred embodiment, the first polypeptide chain comprises the amino acid sequence set forth in SEQ ID NO: 48, and the second polypeptide chain comprises the amino acid sequence set forth in SEQ ID NO: 49; alternatively, the first polypeptide chain comprises the amino acid sequence set forth in SEQ ID NO: 42, and the second polypeptide chain comprises the amino acid sequence set forth in SEQ ID NO: 43; alternatively, the first polypeptide chain comprises the amino acid sequence set forth in SEQ ID NO: 48, and the second polypeptide chain comprises the amino acid sequence set forth in SEQ ID NO: 49; alternatively, the first polypeptide chain comprises the amino acid sequence set forth in SEQ ID NO: 50, and the second polypeptide chain comprises the amino acid sequence set forth in SEQ ID NO: 51. More preferably, the first polypeptide chain comprises the amino acid sequence set forth in SEQ ID NO: 48, and the second polypeptide chain comprises the amino acid sequence set forth in SEQ ID NO: 49.

The present invention further provides an immunoconjugate (e.g., conjugated to a toxin or a small chemical molecule), as well as a pharmaceutical composition and a pharmaceutical combination comprising the single-domain antibody, fusion protein, and bispecific binding molecule of the present invention. In the pharmaceutical composition or pharmaceutical combination, the single-domain antibody, fusion protein, and bispecific binding molecule of the present invention may comprise another therapeutic agent, e.g., another therapeutic agent that may be used for the intended use of the single-domain antibody, fusion protein, and bispecific binding molecule of the present invention, such as a chemotherapeutic agent, a radiotherapeutic agent, an anti-neovascularization molecule, an immunosuppressive drug, an anti-fibrosis drug, a neuroprotective drug, and an anti-tumor molecule.

### IV. Polynucleotide, vector, and host

The present invention provides a nucleic acid encoding any of the above molecules (single-domain antibodies, fusion proteins, and bispecific binding molecules) of the present invention, and also provides a vector comprising the nucleic acid. In one embodiment, the vector is an expression vector. In addition, a host cell comprising the nucleic acid or the vector is provided. In one embodiment, the host cell is eukaryotic. In another embodiment, the host cell is selected from a yeast cell and a mammalian cell (e.g., a CHO cell or a 293 cell). In another embodiment, the host cell is prokaryotic.

In one aspect, the present invention provides a nucleic acid encoding any of the above anti-VEGFC single-domain antibodies or VHH domains. Polypeptides encoded by the nucleic acids may show human VEGFC antigen-binding ability when expressed in a suitable expression vector. In some embodiments, the nucleic acid is operably linked to a nucleic acid encoding another peptide/polypeptide in a reading frame, such that a fusion protein or chimeric polypeptide comprising the single-domain antibody or VHH domain and another peptide/polypeptide is produced when expressed in a suitable expression vector. For ease of production and purification, the single-domain antibody or VHH domain may be fused at the N-terminus to a secretory signal peptide, and/or a tag peptide for facilitating purification, such as a hexahistidine tag. The single-domain antibody or VHH domain may also be fused at the C-terminus to an immunoglobulin Fc portion to form a VHH-Fc antibody.

In a further aspect, the present invention provides a nucleic acid encoding any of the above anti-VEGFA/VEGFC bispecific binding molecules. Polypeptides encoded by the nucleic acids may show human VEGFA and human VEGFC antigen-binding ability when expressed in a suitable expression vector. In one embodiment, the nucleic acid encoding the first polypeptide chain and the second polypeptide chain of the bispecific antibody molecule may be in the same vector or in different vectors. In yet another embodiment, the nucleic acid encoding the first polypeptide chain and the second polypeptide chain of the bispecific antibody molecule may be introduced into the same or different host cells for expression. Thus, in some embodiments, a method for producing the bispecific binding molecule of the present invention comprises the steps of: culturing a host cell comprising a nucleic acid encoding a first polypeptide chain and a second polypeptide chain under conditions suitable for the expression of the first polypeptide chain and the second polypeptide chain of the molecule to produce the bispecific binding molecule of the present invention.

As will be understood by those skilled in the art, each antibody or polypeptide amino acid sequence can be encoded by a variety of nucleic acid sequences because of codon degeneracy.

Nucleic acid sequences encoding the molecules of the present invention may be produced using methods well known in the art, for example by *de novo* solid phase DNA synthesis, or by PCR amplification.

In one embodiment, one or more vectors comprising the nucleic acid of the present invention are provided. In one embodiment, the vector is an expression vector, e.g., a prokaryotic expression vector or a eukaryotic expression vector. The vector includes, but is not limited to, a virus, a plasmid, a cosmid, a λ phage, or a yeast artificial chromosome (YAC).

In one embodiment, provided is a host cell comprising one or more polynucleotides of the present invention. In some embodiments, provided is a host cell comprising the expression vector of the present invention. As used herein, the term "host cell" refers to any kind of cell system that may be engineered to produce the antibody molecules of the present invention. Host cells suitable for replicating and supporting the expression of the antibody molecule of the present invention are well-known in the art. Such cells may be transfected or transduced with a specific expression vector as needed, and a large number of cells comprising vectors may be cultivated and then seeded in a large-scale fermentor, so as to obtain sufficient molecules of the present invention for clinical application. Suitable host cells include prokaryotic microorganisms such as *E*. *coli,* eukaryotic microorganisms such as filamentous fungi or yeast, or various eukaryotic cells such as Chinese hamster ovary cells (CHO) and insect cells. A mammalian cell line suitable for suspension growth may be used. Examples of useful mammalian host cell lines include monkey kidney CV1 line (COS-7) transformed by SV40; human embryonic kidney line (HEK 293 or 293F cells), baby hamster kidney cells (BHK), monkey kidney cells (CV1), African green monkey kidney cells (VERO-76), human cervical cancer cells (HELA), canine kidney cells (MDCK), buffalo rat liver cells (BRL 3A), human lung cells (W138), human liver cells (Hep G2), CHO cells, NSO cells, and myeloma cell lines such as YO, NS0, P3X63 and Sp2/0. For reviews of mammalian host cell lines suitable for protein production, see, e.g., Yazaki and Wu, Methods in Molecular Biology, vol. 248 (edited by B. K. C. Lo, Humana Press, Totowa, NJ), pp. 255-268 (2003). In a preferred embodiment, the host cell is a CHO, HEK293 or NSO cell.

### V. Production and purification of molecules of the present invention

In a further aspect, the present invention provides a method for producing the molecule (single-domain antibody, fusion protein, and bispecific binding molecule) of the present invention, which comprises: culturing a host cell comprising a polynucleotide encoding polypeptide chains under conditions suitable for the expression of the polypeptide chains of the molecule; and assembling the polypeptide chains to produce the molecule under conditions suitable for the assembly of the polypeptide chains into the molecule.

For recombinant production, polynucleotides encoding the polypeptide chains of the molecule of the present invention may be inserted into one or more vectors for further cloning and/or expression in host cells. Methods known to those skilled in the art can be used to construct expression vectors. The expression vector includes, but is not limited to, a virus, a plasmid, a cosmid, a λ phage or a yeast artificial chromosome (YAC). Once the expression vector comprising one or more polynucleotides of the present invention has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, liposome-based transfection, or other conventional techniques.

The antibody molecule prepared as described herein can be purified by known prior art such as high performance liquid chromatography, ion exchange chromatography, gel electrophoresis, affinity chromatography, and size exclusion chromatography. The actual conditions used to purify a particular protein further depend on factors such as net charge, hydrophobicity and hydrophilicity, which would have been apparent to those skilled in the art.

In one embodiment, the single-domain antibody or VHH domain of the present invention may be produced in bacteria, such as *E*. *coli.* In one embodiment, after the single-domain antibody is expressed in the periplasm of the bacteria, it may be isolated and optionally further purified by methods known in the art. In addition, the single-domain antibody, VHH domain, fusion polypeptide, and bispecific molecule may also be expressed in eukaryotic cells. In one embodiment, the cells are 293 cells and are isolated and purified e.g., through a protein A column, after expression.

The purity of the antibody molecule of the present invention can be determined by any one of a variety of well-known analytical methods including size exclusion chromatography, gel electrophoresis, high performance liquid chromatography, and the like. The antibody molecule provided herein can be identified, screened, or characterized for its physical/chemical properties and/or bioactivity through a variety of assays known in the art.

### VI. Assays

The molecule (single-domain antibody, fusion protein, and bispecific molecule) provided herein may be identified, screened, or characterized for physical/chemical properties and/or biological activities through a variety of assays known in the art.

The binding of the molecule of the present invention to human VEGFA and/or human VEGFC may be determined by methods known in the art, such as ELISA, Western blot, and the like, or by the exemplary methods disclosed in the examples herein. For example, the binding kinetics (e.g., K_{D} values) of the molecules may be determined by biological optical interferometry using recombinant VEGFA and/or human VEGFC proteins. In some embodiments, the binding equilibrium dissociation constant, e.g., monovalent binding affinity or bivalent binding affinity, of the molecule for human VEGFA and/or human VEGFC is determined using biological optical interferometry (e.g., Fortebio affinity assay), such as the method shown in Example 3, at a temperature of 30 °C.

VEGFA and/or VEGFC antagonist/inhibitory activity of the molecule of the present invention may be determined by methods known in the art, such as ELISA blocking assays, receptor fluorescent reporter activation assays, cell proliferation assays, or the exemplary methods disclosed in the examples herein. For example, the blocking activity of the molecule to block the binding of human VEGFA and/or human VEGFC to its related receptor may be determined using ELISA blocking assays, such as the method shown in Example 4. The blocking of the molecule on the activation of a VEGFR2 signaling pathway induced by hVEGF-A alone or hVEGF-C alone, or a combination thereof may also be determined by cell-based receptor reporter assays, such as the methods described in Example 5 or 10 using, for example, NFAT-RE-luc2P/KDR HEK293 cells. The inhibition of the molecule on cell survival and/or proliferation induced by hVEGF-A alone or hVEGF-C alone, or a combination thereof *in vitro* may also be determined by cell proliferation assays, e.g., CCK-8 assays, such as the method described in Example 6 or 11 using lymphocytes or endothelial cells.

The anti-neovascularization effect of the molecule of the present invention may be determined by methods known in the art, such as *in vitro* assays and/or *in vivo* animal experiments. For example, *in vitro* endothelial cell tube formation assays, such as the method shown in Example 13, may be used, and VEGFA and VEGF C are utilized to induce the tube formation in human umbilical vein endothelial cells (HUVECs), and the inhibitory effect of the molecule on the VEGF A- and VEGF C-induced tube formation in primary cells is detected. The anti-neovascularization and/or anti-tumor effect of the molecule may also be detected, e.g., in a tumor-bearing mouse model, for example, according to the method shown in Example 14. Alternatively, the inhibition of the molecule on the choroidal neovascularization may be observed, for example, by color fundus photography, fundus fluorescein angiography, or optical coherence tomography scanning after administration of the molecule using, for example, a laser-induced choroidal neovascularization animal model.

### VII. Pharmaceutical composition, pharmaceutical combination, and kit

In one aspect, the present invention provides a composition, e.g., a pharmaceutical composition comprising the molecule (single-domain antibody, fusion protein, or bispecific molecule) of the present invention formulated together with a pharmaceutically acceptable carrier. As used herein, the "pharmaceutically acceptable carrier" includes any and all solvents, dispersion media, isotonic agents and absorption delaying agents, and the like that are physiologically compatible. The pharmaceutical composition of the present invention is suitable for intravenous, intramuscular, subcutaneous, parenteral, rectal, spinal or epidermal administration (e.g., by injection or infusion). In one embodiment, the molecule of the present invention is formulated into a pharmaceutical composition suitable for injection administration. In some embodiments, the molecule of the present invention is formulated into a pharmaceutical composition suitable for intraperitoneal injection. In still other embodiments, the molecule of the present invention is formulated into a pharmaceutical composition suitable for ocular injection administration (e.g., intravitreal administration). In some embodiments, the molecule of the present invention is the only active ingredient in the pharmaceutical composition. In other embodiments, the pharmaceutical composition may comprise the antibody molecule described herein and more than one therapeutic agents.

In another aspect, the present invention further provides a pharmaceutical combination comprising the molecule (single-domain antibody, fusion protein, or bispecific molecule) of the present invention and more than one therapeutic agents.

The therapeutic agents suitable for use in the pharmaceutical composition and the pharmaceutical combination of the present invention may be selected from any one of the following categories: (i) anti-neovascularization drugs; (ii) immunosuppressive drugs; (iii) anti-fibrosis drugs; (iv) neuroprotective drugs; and (v) drugs having an inhibitory effect on tumors.

The compositions of the present invention may be in a variety of forms. These forms include, for example, liquid, semi-solid and solid dosage forms, such as liquid solutions (e.g., injectable solutions and infusible solutions), dispersions or suspensions, liposomes, and suppositories. The preferred form depends on the intended mode of administration and therapeutic use. Commonly preferred compositions are in the form of injectable solutions or infusible solutions. The preferred mode of administration is parenteral (e.g., intravenous, subcutaneous, intraperitoneal (i.p.) and intramuscular) injection. In one preferred embodiment, the antibody molecule is administered by intravenous infusion or injection. In another preferred embodiment, the antibody molecule is administered by intramuscular, intraperitoneal or subcutaneous injection.

As used herein, the phrases "parenteral administration" and "administered parenterally" mean modes of administration other than enteral and topical administration, typically by injection, and include, but are not limited to, intravenous, intramuscular, intra-arterial, intradermal, intraperitoneal, transtracheal, subcutaneous injection and infusion.

Therapeutic compositions generally should be sterile and stable under the conditions of manufacture and storage. The compositions can be prepared into solutions, microemulsions, dispersions, liposomes, or lyophilized forms. Sterile injectable solutions can be prepared by adding a required amount of an active compound (i.e., antibody molecule) to a suitable solvent, and then filtering and disinfecting the resulting mixture. Generally, dispersions are prepared by incorporating the active compound into a sterile vehicle, which comprises a basic dispersion medium and other ingredients. Coating agents such as lecithin can be used. In the case of dispersions, the proper fluidity of a solution can be maintained by using a surfactant. Prolonged absorption of injectable compositions can be caused by including in the compositions a substance that delays absorption such as monostearate and gelatin.

The pharmaceutical composition of the present invention may comprise a "therapeutically effective amount" or a "prophylactically effective amount" of the molecule of the present invention. "Therapeutically effective amount" refers to an amount effective to achieve a desired therapeutic result at a necessary dose for a necessary period of time. The therapeutically effective amount can be varied according to a variety of factors such as disease state, age, gender, and weight of the individual. The therapeutically effective amount is an amount in which any toxic or harmful effect is outweighed by the therapeutically beneficial effect. The "therapeutically effective amount" preferably inhibits a measurable parameter (e.g., tumor growth rate) by at least about 20%, more preferably at least about 40%, even more preferably at least about 60%, and still more preferably at least about 80%, relative to untreated subjects. The ability of the molecule of the present invention to inhibit a measurable parameter (e.g., tumor volume) may be evaluated in an animal model system that predicts efficacy in human tumors.

"Prophylactically effective amount" refers to an amount effective to achieve a desired prophylactic result at a necessary dose for a necessary period of time. Generally, since a prophylactic dose is used in subjects before or at an earlier stage of a disease, the prophylactically effective amount will be less than the therapeutically effective amount.

A kit comprising the antibody molecule described herein is also within the scope of the present invention. The kit may include one or more other elements, including, for example, a package insert; other reagents, such as a label or a reagent for coupling; a pharmaceutically acceptable carrier; and a device or other materials for administration to a subject.

### VIII. Use and method of molecule of the present invention

In one aspect, the present invention provides *in vivo* and *in vitro* use and methods for the use of the molecule of the present invention.

In some embodiments, the use and the method of the present invention involve the application of the molecule of the present invention *in vivo* and/or *in vitro* in:
- binding to a VEGFA antigen and/or a VEGFC antigen;
- blocking the binding of VEGFA and/or VEGFC to its related receptor, such as VEGFR2 and/or VEGFR3;
- inhibiting the activation of the VEGFR2 and/or VEGFR3 cell signaling induced by VEGFA and/or VEGFC;
- inhibiting VEGFA- and/or VEGFC-induced vascular endothelial cell survival, proliferation, and/or migration;
- inhibiting VEGFC-induced lymphocyte survival and/or proliferation;
- inhibiting VEGFC-induced lymphangiogenesis and lymphatic endothelial cell growth and migration;
- inhibiting VEGFA- and/or VEGFC-induced neovascularization and/or vascular leakage; and
- inhibiting neovascularization and/or growth and/or metastasis of tumors.

In some embodiments, the antibody molecule of the present invention or the pharmaceutical composition comprising the antibody molecule of the present invention is used as a drug for the treatment and/or prevention of a disease in an individual or as a diagnostic tool for a disease; preferably, the individual is a mammal, and more preferably a human.

In some embodiments, the present invention provides a method for treating a neovascularization-associated disease, comprising administering to a subject the molecule (the single-domain antibody polypeptide or the fusion protein or bispecific binding protein thereof) of the present invention, or a pharmaceutical composition thereof. In some embodiments, the disease is a solid tumor, preferably melanoma, and administration of the bispecific binding protein inhibits neovascularization in the tumor and/or tumor growth. In other embodiments, the disease is an ocular disease, preferably age-related macular degeneration, diabetic retinopathy, retinal vascular occlusion, and corneal neovascularization.

In some embodiments, the present invention provides use of the single-domain antibody polypeptide of the present invention or the fusion protein thereof, or the bispecific binding molecule of the present invention in the manufacture of a medicament for the treatment and/or prevention of a disease in a subject and/or in the manufacture of a diagnostic tool for the diagnosis of a disease, wherein the disease is preferably a neovascularization-associated disease, such as a solid tumor and an ocular disease.

In any of the above embodiments, one or more additional active agents, e.g., chemotherapeutic agents and/or cancer therapeutic agents or anti-neovascularization therapies, may be further administered in combination with the single-domain antibody polypeptide or bispecific binding protein of the present invention. The form of the combined administration may be simultaneous administration, concurrent administration, or sequential administration in any order.

In a further aspect, the present invention provides a diagnostic method for detecting the presence of related antigens in a biological sample, such as a serum, semen, urine or tissue biopsy sample (e.g., from a hyperproliferative or cancerous lesion), *in vitro* or *in vivo.* The diagnostic method comprises: (i) contacting a sample (and optionally a control sample) with the (labeled or unlabeled) molecule (single-domain antibody, fusion protein, or bispecific binding molecule) of the present invention or administering the antibody molecule to a subject under conditions that allow interactions, and (ii) detecting the formation of a complex between the molecule and the sample (and optionally the control sample). The formation of a complex indicates the presence of the related antigen, and in some cases, it may show the suitability of or need for the treatment and/or prevention described herein.

### EXAMPLES

### Example 1. Preparation of Phage Immune Library

### Construction of alpaca immune or synthetic library

1.1 Two healthy adult alpacas (Chengdu NBbiolab, Co. Ltd.) were selected, 0.5 mg of recombinant protein antigen VEGF C (Beijing Sino Biological) and Freund's adjuvant were mixed homogeneously according to a ratio of 1:1, and the alpacas were immunized by subcutaneous injection at multiple sites on the back for four times with an immune interval of 2 weeks.
1.2 Alpaca peripheral blood (50 mL) was collected, lymphocytes were isolated, 1 mL of Trizol reagent (THERMO) was added per 2.5 × 10⁷ viable cells, and the total RNA was extracted using a chloroform/isopropanol precipitation method. Reverse transcription was performed using PrimeScript reverse transcription kit (Takara) by taking 10 µg of RNA as a template. VHH encoding nucleic acids were produced by PCR amplification using cDNA as a template, followed by insertion into a phage display vector that would express and display a fusion polypeptide of VHH fragments with Flag tags and hexahistidine tags and a phage gpIII protein; E. *coli* TG1 competent cells were transformed with the display vector to construct a VHH antibody library.

Briefly, the first round and second round of PCR reactions were performed using cDNA as a template (see J Immunol Methods., 2007 July 31; 324(1-2): 13-25 for the specific method). The pC3-HF vector and the product from the second round of PCR were subjected to double digestion using SacI and SalI (Thermo), respectively, the digested products were reacted with T4 ligase (Thermo), and TG1 competent cells were electrotransformed to construct a VHH antibody library. The bacterial liquid was cryopreserved at -80 °C.

The thawed bacterial liquid was inoculated into 100 mL of YT-AG medium (Shanghai Sangon Biotech), a helper phage was added for infection, and the bacterial cell pellet was resuspended with 2× YT-AK medium (Shanghai Sangon Biotech) and cultured at 37 °C and at 200 rpm overnight. The culture supernatant was collected, and a recombinant phage was prepared using a PEG/NaCl precipitation method.

1.3 The recombinant phage was subjected to 3 rounds of panning experiments using the biotin-labeled antigen VEGFC (Beijing Sino Biological). 50 µL of Dynabeads^{™} M280 magnetic beads (Thermo) and an appropriate amount of biotin-labeled antigens were added to each tube, and the mixture was incubated at room temperature for 30 min. 1 × 10¹² cfu of recombinant phage was added, and the mixture was incubated at room temperature for 1 h. The resulting mixture was washed 10 times with 1 mL of PBST, each time for 5 min. Finally, 0.5 mL of glycine buffer with pH of 2.5 was added, the antigen-bound recombinant phage was eluted, TG1 was infected, and the cells were cultured overnight. After the infection by the helper phage, the recombinant phage was prepared for the next round of panning experiment, and TG1 bacterial clones with positive VHHs were identified.

1.4 The binding activity was detected by Binding ELISA and the clones were sequenced.

The VEGFC antigen (Beijing Sino Biological) was taken in advance and diluted to 0.5 µg/mL with PBS buffer to coat a 96-well ELISA plate, and the plate was stored in a refrigerator at 4 °C overnight. The antigen-coated plate was washed 3 times with PBST, a blocking reagent was added to 300 µL/well, and the plate was left to stand at room temperature for 1 h. The plate was washed 3 times with PBST, 80 µL of blocking reagent and 20 µL of expression supernatant of the TG1 strain with positive VHHs identified in step 1.3 described above were added, and the plate was shaken at room temperature for 1 h.

The plate was washed 3 times with PBST, 100 µL of Anti-Flag/HRP secondary antibody (Sigma) diluted with the blocking reagent was added per well, and the plate was shaken at room temperature for 40 min. The plate was washed 6 times with PBST, TMB chromogenic solution was added to 100 µL/well, and the color development was performed in the dark for 5-15 min. Then 100 µL/well of stop solution was added. The plate was read on a microplate reader, and the absorbance values at OD450 nm were measured. Bacterial clones with a reading value greater than 0.5 were selected and sent to Genewiz for sequencing. A single clone of TG1 strain containing each corresponding VHH sequence was selected, and glycerol was added. The mixture was cryopreserved in a refrigerator at -80 °C.

### Example 2. Production and Purification of Prokaryotic Antibody

The obtained TG1 single clones containing positive VHHs were used to be expressed and purified to obtain VHH antibody proteins.

The TG1 strain containing VHH expression plasmids identified in Example 1 was inoculated into 800 mL of LB-Amp medium and cultured at 37 °C and at 200 rpm until the OD600 value was 0.5-0.6. 1 mM IPTG was added to the bacterial liquid to induce the expression of the VHH fragments, and the mixture was cultured at 28 °C and at 200 rpm overnight. The culture supernatant was collected. After centrifugation, 15 mL of PB and 1 mg/mL of polymyxin were added to resuspend the bacteria, and the bacterial periplasm was lysed. The mixed solution was centrifuged again and filtered through a 0.22 µm filter membrane. The periplasm lysate was passed through a 1 mL Ni Sepharose pre-column, and the column was washed 2 times with PBS. The target protein was eluted with 0.5 M imidazole, and the protein concentration was measured by an ultraviolet method. The protein concentration of the eluted target protein was measured by the ultraviolet method, and the eluted target protein was aliquoted into multiple tubes and stored in a refrigerator at -40 °C.

Two anti-VEGF C VHH antibodies (LA49G9 and LA63G12) sharing the following HCDR motifs were obtained by alpaca VHH library screening:
HCDR1: GSXFSXYAMG;
HCDR2: ATTSGGSTLYADSVKG; and
HCDR3: XWRGSDPENY

On the basis of the 2 VHH antibodies obtained, humanized VHH antibodies (LA49G9.2 and LA63G12.1) and affinity-matured humanized VHH antibodies (atn63G12-14B11 and am63G12-5G8-18B9) were produced.

The CDR amino acid sequences and VHH amino acid sequences of the VHH antibodies of the present invention, as well as the sequence numbers are shown in the sequence list and FIG. 19.

In the VHH antibody biological activity assays of the following examples, humanized LA49G9.2 and LA63G12.1 molecule assays were performed using dimerized VHH-Fc antibodies, the remainder being performed using VHH single-domain antibodies.

### Example 3. Binding Kinetics of Antibodies of the Present Invention for Antigens as Determined by Bio-Layer Interferometry

The equilibrium dissociation constant (KD) for binding of the antibodies of the present invention to human VEGF C was determined by bio-layer interferometry (ForteBio). A ForteBio affinity assay of prior art was performed (Estep, P., et al., "High throughput solution Based measurement of antibody-antigen affinity and epitope binning", MAbs, 2013.5(2): 270-8).

Half an hour before the experiment, an appropriate number of AMQ (Pall, 1506091) (for sample detection) or AHQ (Pall, 1502051) (for positive control detection) sensors depending on the number of samples were soaked in SD buffer (PBS 1×, BSA 0.1%, Tween-20 0.05%).

100 µL of SD buffer, antibodies, and antigens (human VEGF C, Beijing Sino Biological) were added to a 96-well black polystyrene half-area microplate (Greiner, 675076). The sensors were arranged according to the positions of the samples. The instrument settings were as follows: the operation procedures were Baseline, Loading -1 nm, Baseline, Association, and Dissociation; the run time of each procedure was dependent on the rates of association and dissociation; the rotation speed was 400 rpm, and the temperature was 30 °C. The K_{D} values were analyzed by ForteBio analysis software.

In the experiments performed by the assays described above, the affinities of the antibodies are shown in Table 1:

**Table 1. Affinity constants (equilibrium dissociation constants) for monovalent bindings of antigens and antibodies by ForteBio assay**

| Antibody | Antigen | K_{D} (M) | Kₒₙ (1/Ms) | k_{dis} (1/s) |
|---|---|---|---|---|
| LA49G9 | VEGFC | 1.00E-08 | 5.43E+04 | 5.44E-04 |
| LA49G9.2 | | / | / | / |
| LA63G12 | | 1.64E-08 | 4.65E+04 | 7.61E-04 |
| LA63G12.1 | | / | / | / |
| Am63G12-14B11 | | 6.21E-09 | 5.53E+04 | 3.43E-04 |
| Am63G2-5G8-18B9 | | 5.70E-09 | 6.31E+04 | 3.60E-04 |

**Table 2. Affinity constants (equilibrium dissociation constants) for bivalent bindings of antigens and antibodies by ForteBio assay**

| Antibody | Antigen | K_{D} (M) | Kₒₙ (I/Ms) | k_{dis} (1/s) |
|---|---|---|---|---|
| LA49G9 | VEGFC | 6.13E-10 | 3.26E+05 | 2.00E-04* |
| LA49G9.2 | | 6.45E-10 | 3.10E+05 | 2.00E-04* |
| LA63G12 | | 6.56E-10 | 3.05E+05 | 2.00E-04* |
| LA63G12.1 | | 6.22E-10 | 3.22E+05 | 2.00E-04* |

| | | | | |
|---|---|---|---|---|
| denotes dissociation constants exceeding ForteBio detection limits | | | | |

In the above assays, the monovalent KD values of the antibodies LA49G9 and LA63G12 for human VEGFC are 1.00E-08 M and 1.64E-08 M, respectively; the binding affinities of the affinity-matured antibodies Am63G12-14B11 and Am63G2-5G8-18B9 are further improved, and the monovalent KD values are 6.21E-09 M and 5.70E-09 M, respectively. The antibodies LA49G9, LA49G9.2, LA63G12, and LA63G12.1 have KD values of 6.13E-10 M, 6.45E-10 M, 6.56E-10 M, and 6.22E-10 M for bivalent affinity with human VEGFC, respectively.

### Example 4. ELISA Blocking Assay of Anti-VEGFC VHH Antibodies

The blocking effects of the antibodies of the present invention on the binding of hVEGFC to a receptor KDR were determined.

SA (streptavidin) was diluted to 1 µg/mL, and 100 µL/well of SA was plated in a microplate at 4 °C overnight. The plate was washed 3 times with PBST and blocked with 3% BSA for 1.5 h. The plate was washed 3 times with PBST, 50 ng/mL of VEGFC-biotin (biotin-labeled human VEGF C) was added, and the plate was incubated for 1.5 h. 50 µL of antibody was incubated with VEGFR2-Fc (with a final concentration of 0.2 µg/mL, Beijing Sino Biological) or VEGFR3-Fc (with a final concentration of 0.2 µg/mL, Beijing Sino Biological) for 20 min in advance, and the mixture was added to the plate. The plate was washed 3 times with PBST, an anti-human FcHRP antibody (diluted in a ratio of 1:10000, BETHYL) was added, and then the plate was incubated for 30 min. The plate was washed 6 times with PBST, the color development was performed with TMB (SOLARBIO) for 5 min, and OD450 nm was read after stopping.

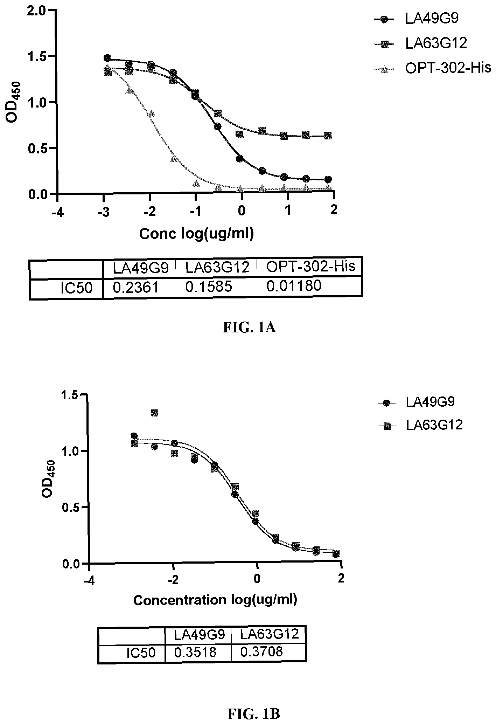

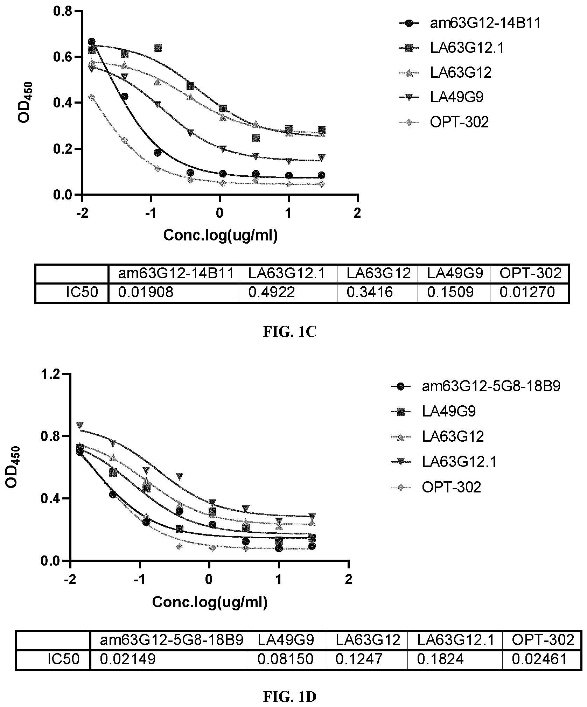

The blocking results of the anti-VEGF VHH antibodies obtained in the present invention are shown in FIG. 1. The candidate molecules LA49G9 and LA63G12, the humanized antibody LA63G12.1, and the affinity-matured molecules Am63G12-14B11 and Am63G2-5G8-18B9 were all capable of blocking the binding of VEGFC to VEGFR2 and VEGFR3, and both affinity-matured molecules had substantially comparable blocking IC50 values to the positive control molecule OPT-302 (VEGF-C-trap, SEQ ID NO: 52).

### Example 5. Assay for Blocking of VEGFC-Induced HEK293 KDR Reporter Activation by Anti-VEGFCVHH Antibodies

VEGFC may bind to a related receptor VEGFR2 (KDR), activate a VEGFR2 signaling pathway, and induce the survival, proliferation, and migration of vascular endothelial cells. This research utilized a KDR reporter assay system and used NFAT-RE-luc2P/KDR HEK293 cells (Promega, Cat. CS181401) to detect the blocking effects of the gradiently diluted antibodies on a VEGFC activation-related receptor signaling pathway.

### Experimental procedures referred to supplier's instructions:

NFAT-RE-luc2P/KDR HEK293 cells which were changed into an experimental medium (DMEM medium containing 10% FBS) 3 days in advance were taken out, and the old medium was removed by pipetting. The medium was washed once with PBS, then 1 mL of accutase solution (Sigma) was used to digest the cells until the cells became round and were detached from the wall, and 5 mL of diluted medium (DMEM medium containing 10% FBS) was used to stop the reaction. The cells were pipetted into a centrifuge tube and centrifuged at 1000 rpm for 5 min, the medium was discarded, 10 mL of diluted medium was added to resuspend the cells, and the cells were mixed homogeneously and counted. The cell viability should be more than 90%. The cell density was adjusted to 0.8 × 10⁶ cells/mL with the diluted medium, and the mixture was added to a 96-well white cell culture plate at 50 µL/well according to an experimental layout.

A mixed solution of hVEGFC (R&D) with a concentration of 200 ng/mL and the gradiently diluted antibodies to be tested (including the VHH antibodies of the present invention, positive control molecule (OPT-302), and negative control antibody (IgG isotype control antibody)) was prepared, and the mixture was left to stand for 30 min, and then added to a 96-well white cell culture plate containing cells at 50 µL/well. The plate was put into a 37 °C incubator with 5% carbon dioxide and incubated for 6 h. A blank control group without any antibody and VEGFC as well as a VEGF-C experimental group with VEGFC only and without any antibody were set at the same time.

The 96-well white cell culture plate incubated for 6 h was taken out from the carbon dioxide incubator, and equilibrated for 10-15 min to room temperature. The Bio-Glo Luciferase Assay System (Promega) equilibrated to room temperature in advance was added to the 96-well white cell culture plate at 100 µL/well according to the experimental layout, and the plate was incubated at room temperature for 5 min in the dark.

Fluorescence values were read using a multimode microplate reader, the chemiluminescence mode was selected for plate reading mode, the end point method was selected for plate reading type, and the wavelength was set to full wavelength. The fluorescence was collected column by column, and the collection time for each column was 1000 ms.

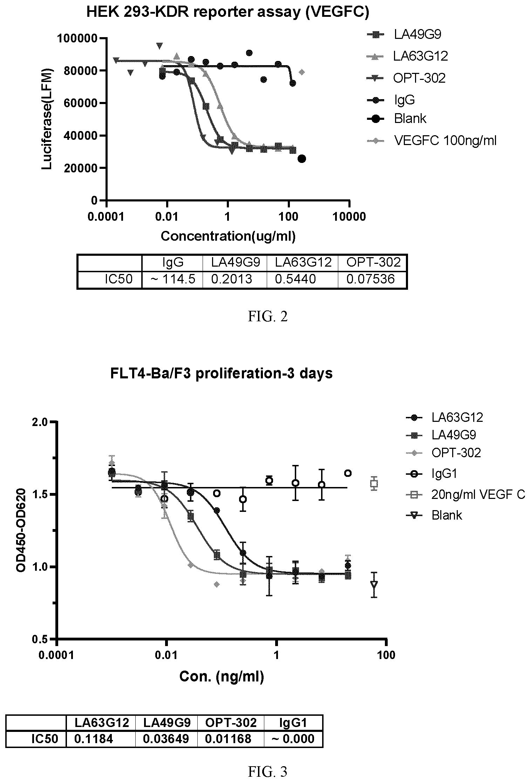

The results are shown in FIG. 2. LA63G12 and LA49G9 could completely inhibit the activation of the KDR signaling pathway induced by VEGFC.

### Example 6. Assay for Inhibition of VEGFC-Induced BaF3-FLT4 Cell Proliferation by Anti-VEGFC VHH Antibodies

In this research, BaF3 cells overexpressing FLT4 (VEGFR3), BaF3-FLT4 (Institute of Basic Medical Sciences, Chinese Academy of Medical Sciences), were co-incubated with the antibodies and a recombinant human VEGFC protein. The number of viable cells was detected by the CCK-8 kit (Dojindo), thereby reflecting the inhibitory effects of different antibodies on the VEGFC-induced BaF3-FLT4 proliferation.

BaF3 cells were infected with a lentivirus carrying the FLT4 gene to obtain the BaF3 cells overexpressing FLT4, BaF3-FLT4.

The inhibitory assays for cell proliferation were performed according to instructions of the CCK-8 kit. The experimental medium was prepared using a 1640 medium containing 10% FBS. The test antibodies (including the VHH antibodies of the present invention, positive control molecule (OPT-302), and negative control antibody (IgG isotype control antibody)) with the maximum final concentration of 20 µg/mL were serially diluted in a ratio of 1:3. A blank control group without any antibody and VEGFC as well as a VEGF-C experimental group with VEGFC only and without any antibody were set at the same time. In the test system, the final concentration of hVEGFC (R&D) was 20 ng/mL, and the final concentration of BaF3-FLT4 cells was 2 × 10⁵ cells/mL. The system was added to a 96-well plate at 100 µL per well, and the plate was incubated in a CO₂ incubator at 37 °C for 72 h. Thereafter, 15 µL of CCK-8 was added per well, and the plate was incubated in the CO₂ incubator at 37 °C for 4 h. The absorbance value was measured by adopting dual wavelength, the detection wavelength was 450 nm, and the reference wavelength was 620 nm. OD450-OD620 values were measured.

The results are shown in FIG. 3. The antibodies LA63G12 and LA49G9 of the present invention could both effectively inhibit hVEGFC-induced BaF3-FLT4 survival and proliferation *in vitro.*

### Example 7. Anti-VEGFC VHH Antibody Humanization, Activity Assay, and Protein Expression Purification

The LA49G9 and LA63G12 antibodies were humanized by the following steps:
(1) determining a CDR loop structure;
(2) searching a human germline sequence database for the closest homologous sequences for each V/J region;
(3) screening human germlines for the highest match in a heavy chain and a minimum quantity of back mutations;
(4) constructing the CDR regions of the chimeric antibody onto the framework regions of a human antibody;
(5) determining amino acid positions that maintained CDR functions in the framework regions based on the sequences and structural features;
(6) adding back mutations (back to the input amino acids) at important sequence positions identified; and
(7) optimizing amino acids at risk sites.

The humanized antibodies LA49G9.2 and LA63G12.1 were obtained and then sequenced.

Plasmids containing nucleic acids encoding the anti-VEGFC antibodies were prepared for the transfection of 293 cells and expression of VHH single-domain antibodies or VHH-Fc antibodies. Expi-293 cells (Invitrogen) were passaged according to a desired transfection volume. The cell density was adjusted to 1.5 × 10⁶ cells/mL the day before transfection. The cell density on the day of transfection was approximately 3 × 10⁶ cells/mL. An appropriate amount of plasmids was added to F17 culture medium (Gibco, A13835-01) at 1/10 of the final volume as the transfection buffer, and mixed. An appropriate amount of polyethylenimine (PEI) (Polysciences, 23966) was added to the plasmids (the ratio of plasmids to PEI was 1:3 in the 293F cells), mixed and incubated at room temperature for 10 min, resulting in a DNA/PEI mixture. After resuspension of the cells in a DNA/PEI mixture, the cells were incubated at 36.5 °C with 8% CO₂ for 24 h, and FEED (Sigma) at 2% of the transfection volume was added. The cells were incubated at 36.5 °C with 8% CO₂ at 120 rpm. On Day 6 days of subculture or until viability fell below 60%, the cell supernatant was collected and purified.

Before purification, the collected cultures were centrifuged at 4500 rpm for 30 min, and the cells were discarded. The supernatant was filtered through a 0.22 µL filter. Protein A column (Hitrap Mabselect Sure 5 × 5 mL, GE, 11-0034-95) was equilibrated with 10 mL of binding buffer (20 mM sodium phosphate, 150 mM NaCl, pH 7.0). The filtered supernatant was loaded to the purification column, which was then re-equilibrated with 15 mL of binding buffer. 5 mL of eluent buffer (citric acid + sodium citrate 0.1 M, pH 3.5) was added. The eluate was collected, and 80 µL of Tris-HCl was added per mL of eluate. The collected antibodies was changed into PBS (Gibco, 70011-044) by ultrafiltration concentration, and the concentrations were measured.

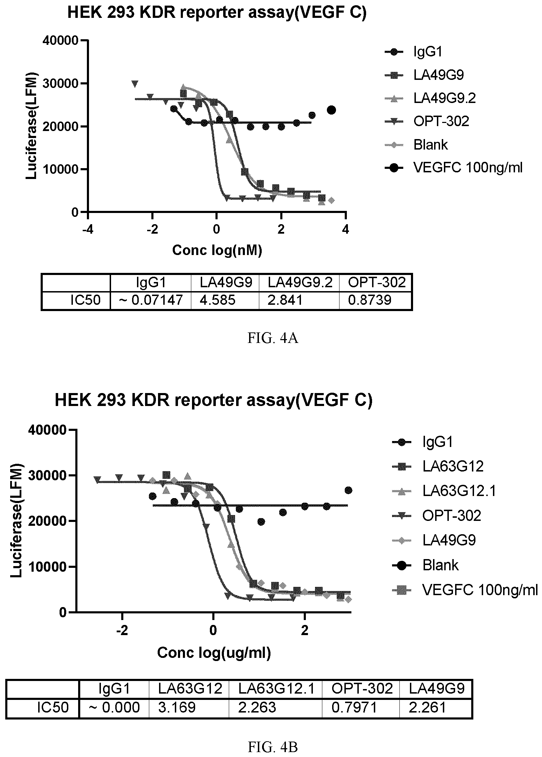

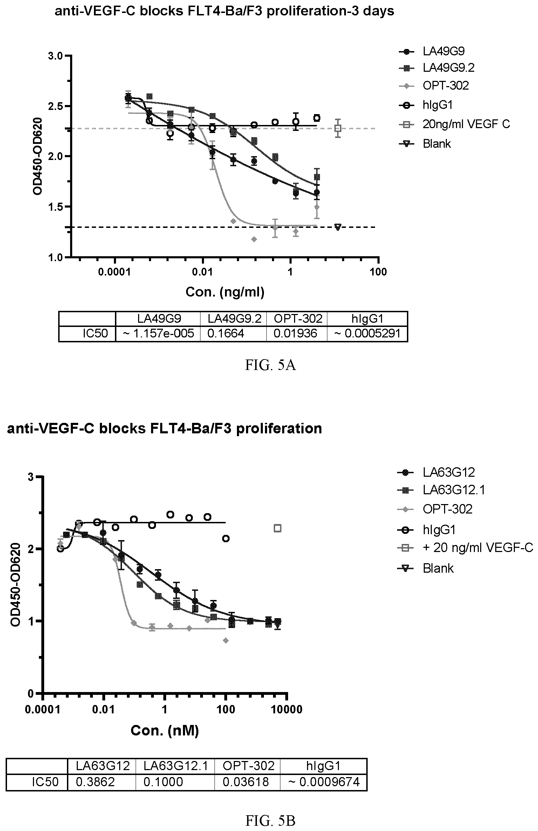

The activity of the humanized antibodies was determined according to the method described in Examples 3-6. The results are shown in Tables 1 and 2 above and in FIGs. 1, 4, and 5, respectively. The results show that the humanized antibodies had comparable antigen hVEGF C binding activity and VEGFR2/VEGFR3 receptor blocking activity to the parent antibody; the humanized antibodies blocked the activation of the VEGFR2 signaling pathway induced by VEGF C in cell-based assays (FIG. 4), and blocked the proliferation of Baf3-FLT4 cells induced by the binding of VEGFC to VEGFR3 (FIG. 5).

### Example 8. Anti-VEGF C VHH Affinity Maturation

The LA63G12.1 humanized single-domain antibody gene was selected as a template, amino acid random mutations were introduced to the antigen-binding regions (CDRs), and degenerate primers (Genewiz) containing NNK codons and primers specific to the framework regions were designed and synthesized. The antibody mutant gene library was amplified by overlap extension PCR (OE-PCR). PCR fragments and vectors were digested, ligated, and transformed with TG1 strains by the same method as that in Example 1 to prepare a recombinant phage library, which was subjected to 3 rounds of phage panning. Prokaryotic protein expression, ELISA assay for clone binding activity, and sequencing analysis were performed according to Example 2. Finally, 2 mutants with significantly improved affinity were selected, and clone numbers were atn63G12-14B11 and am63G12-5G8-18B9. Eukaryotic VHH single-domain antibody expression was performed according to Example 7.

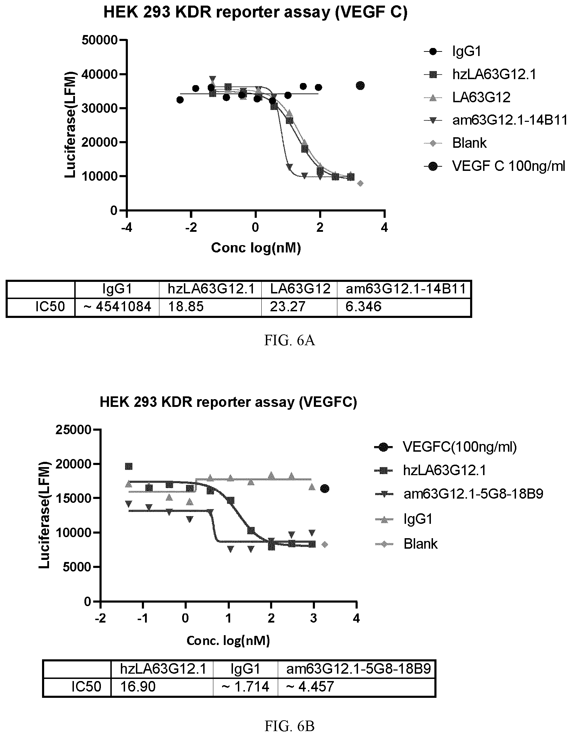

The affinity-matured VHH assay method was performed as described in Example 4, Example 5, and Example 6. The results are shown in FIGs. 1, 6, and 7, respectively. The results show that the affinity-matured antibodies had further improved biological activity against VEGFC compared to the parent antibody.

### Example 9. Construction of Anti-VEGFA/VEGF C Bispecific Antibodies

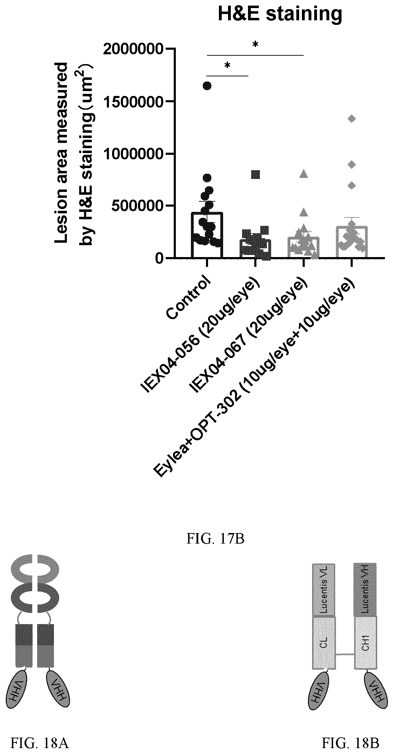

Anti-VEGFA/VEGFC bispecific antibodies were constructed from LA63G12, LA63G12.1, am63G12-14B11, and am63G12-5G8-18B9, and eukaryotic protein expression was performed in 293 cells according to Example 7. Briefly:
The bispecific antibody comprising a first polypeptide chain and a second polypeptide chain as shown in FIG. 18A was constructed by linking a VHH single-domain antibody to the C-terminus of an Fc region of VEGF-Trap (Aflibercept, Eylea) via a linker. VEGF-Trap is a recombinant fusion protein (SEQ ID NO: 25) consisting of VEGF-A binding domain portions (SEQ ID NO: 26) from human VEGF receptors 1 and 2 fused at the C-terminus to a human IgG1 Fc region (SEQ ID NO: 27). VEGF-Trap is in the form of a dimer, thereby providing high affinity for VEGF_A binding and blocking the activation of the VEGFR signaling pathway induced by VEGF_A.

The bispecific antibody comprising a first polypeptide chain and a second polypeptide chain as shown in FIG. 18B was constructed by linking a VHH single-domain antibody to the C-terminus of a Fab portion of an anti-VEGF_A antibody Lucentis (ranibizumab) via a linker. Lucentis is a recombinant humanized IgG1 kappa isotype monoclonal antibody, wherein a VH-CH1 polypeptide chain having an amino acid sequence set forth in SEQ ID NO: 28 and a VL-CL polypeptide chain having an amino acid sequence set forth in SEQ ID NO: 29 are paired to form a Fab, which binds to human VEGF-A and inhibits the biological activity of human VEGF-A.

The bispecific antibodies constructed and their compositions are listed in Table 3 below.

| Bispecific antibody | Anti-VEGF-A component | Linker | Anti-VEGF-C component | First/second polypeptide chain sequences constituting antibodies |
|---|---|---|---|---|
| IEX04-026 | Aflibercept | G(G₄S)₃ | LA63G12 | SEQ ID NOs: 40/40 |
| IEX04-037 | Aflibercept | G(G₄S)₃ | LA63G12.1 | SEQ ID NOs: 41/41 |
| IEX04-039 | Lucentis Fab | (G₄S)₃ | LA63G12.1 | SEQ ID NOs: 42/43 |
| IEX04-041 | Aflibercept | G(G₄S)₂ | LA63G12.1 | SEQ ID NOs: 44/44 |
| IEX04-042 | Aflibercept | G(G₄S)₄ | LA63G12.1 | SEQ ID NOs: 45/45 |
| IEX04-046 | Aflibercept | G(G₄S)₃ | am63G12.1-14B11 | SEQ ID NOs: 46/46 |
| IEX04-056 | Aflibercept | G(G₄S)₃ | am63G12.1-5G8-18B9 | SEQ ID NOs: 47/47 |
| IEX04-067 | Lucentis Fab | (G₄S)₃ | am63G12.1-14B11 | SEQ ID NOs: 48/49 |
| IEX04-069 | Lucentis Fab | (G₄S)₃ | am63G12.1-5G8-18B9 | SEQ ID NOs: 50/51 |

### Example 10. Assays for Blocking of VEGFA- or VEGFC-Induced HEK293 KDR Reporter Activation by Anti-VEGFA/VEGF C Bispecific Antibodies

VEGFA or VEGF C may bind to a related receptor VEGFR2 (KDR), activate a VEGFR2 signaling pathway, and induce the survival, proliferation, and migration of vascular endothelial cells. This research utilized a KDR reporter assay system and used NFAT-RE-luc2P/KDR HEK293 cells (Promega, Cat. CS181401) to detect the blocking effects of the gradiently diluted antibodies on VEGFA and VEGF C activation-related receptor signaling pathways. Experimental procedures were substantially performed as described in Example 5. Briefly, 96-well white cell culture plates containing NFAT-RE-luc2P/KDR HEK293 cells (0.8 ×10° cells/mL, 50 µL/well) were prepared. A mixed solution of hVEGFA (R&D) with a concentration of 100 ng/mL or hVEGFC (R&D) with a concentration of 200 ng/mL and the gradiently diluted antibody to be tested was prepared and left to stand for 30 min. 50 µL/well of the mixed solution was added to the 96-well white cell culture plate containing NFAT-RE-luc2P/KDR HEK293 cells, and the plate was put into a 37 °C incubator with 5% carbon dioxide and incubated for 6 h. A blank control group without any antibody and VEGFC, an experimental group with VEGFC only and without any antibody, and an experimental group with VEGFC and IgG isotype control antibody were set at the same time. In addition, as a comparison, the blocking activity of the anti-VEGF A molecule IBI304 (SEQ ID NO: 53) and the bispecific antibody Faricimab (anti-Ang-2/anti-VEGF-A) on the VEGF-A signaling pathway was detected. The blocking activity of the anti-VEGF-C molecule OPT-302 on the VEGF-C signaling pathway was also detected.

The 96-well white cell culture plate incubated for 6 h was taken out from the carbon dioxide incubator, and equilibrated for 10-15 min to room temperature. The Bio-Glo Luciferase Assay System (Promega) equilibrated to room temperature in advance was added to the 96-well white cell culture plate at 100 µL/well according to the experimental layout, and the plate was incubated at room temperature for 5 min in the dark.

Fluorescence values were read using a multimode microplate reader, the chemiluminescence mode was selected for plate reading mode, the end point method was selected for plate reading type, and the wavelength was set to full wavelength. The fluorescence was collected column by column, and the collection time for each column was 1000 ms.

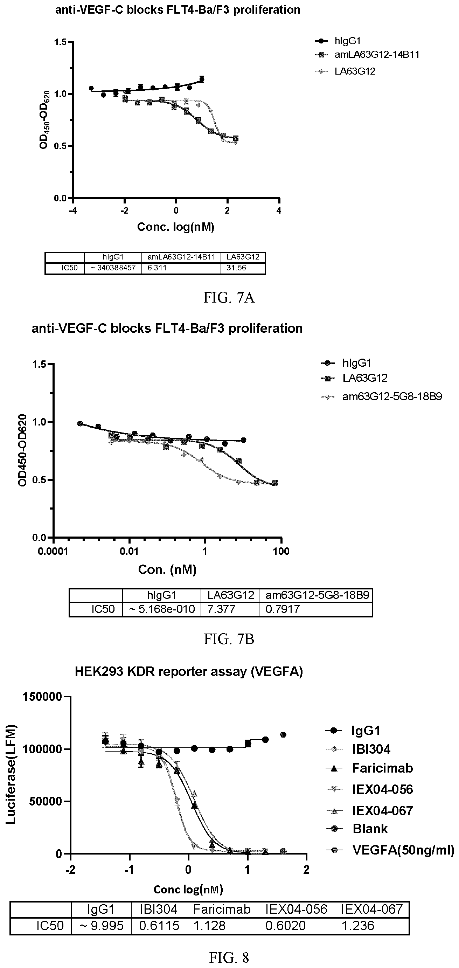

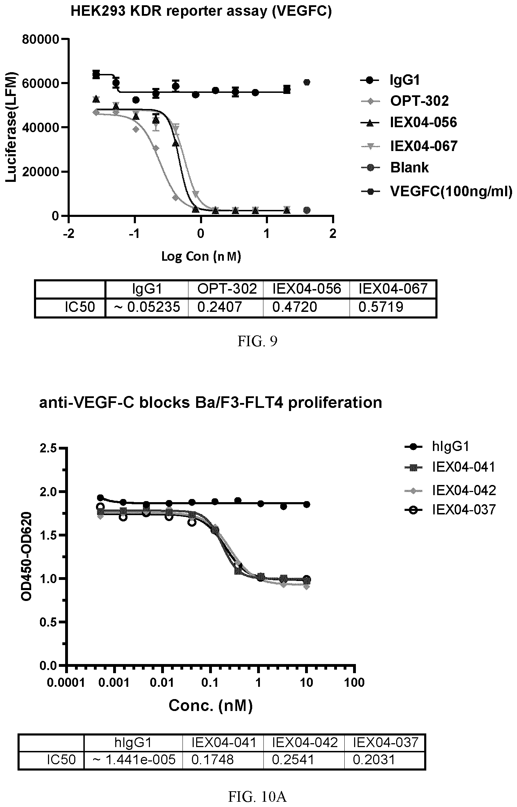

The assay results are shown in FIG. 8 and FIG. 9. Both the anti-VEGFA and anti-VEGFC bispecific antibodies tested could block the activation of the KDR signaling pathway induced by VEGFA (FIG. 8) or VEGFC (FIG. 9).

### Example 11. Assays for Inhibition of VEGFC-Induced BaF3-FLT4 Proliferation by Anti-VEGFA/VEGF C Bispecific Antibodies

The above bispecific antibodies of the present invention were applied to a BaF3-FLT4 proliferation experimental system to detect the effects of the antibodies on VEGFC-induced BaF3-FLT4 proliferation. Experimental procedures were substantially performed as described in Example 6.

The experimental medium was prepared using a 1640 medium containing 10% FBS. The test antibodies had a maximum final concentration of 10 nM and were serially diluted in a ratio of 1:3. In the test system, the final concentration of hVEGFC (R&D) was 20 ng/mL, and the final concentration of BaF3-FLT4 cells was 2 × 10⁵ cells/mL.

In the assay, a blank control group without any antibody and VEGFC, an experimental group with VEGFC only and without any antibody, and an experimental group with VEGFC and IgG isotype control antibody were set at the same time. In addition, as a comparison, the inhibitory activity of the anti-VEGF-C molecule OPT-302 on the VEGFC-induced BaF3-FLT4 cell proliferation was detected.

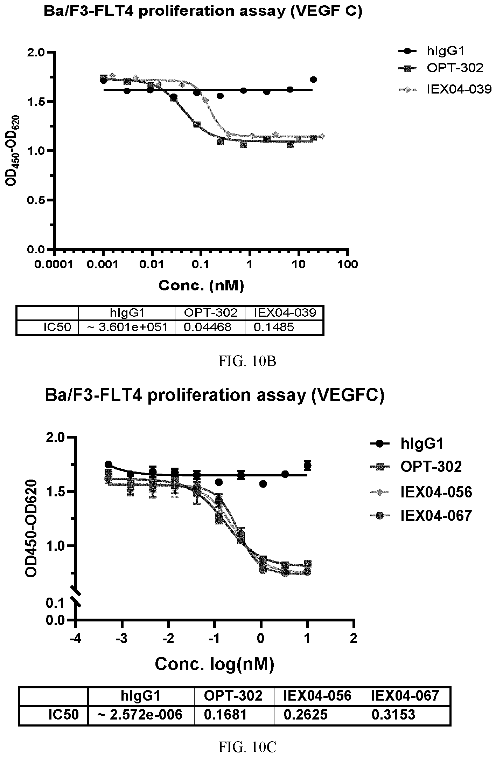

The assay results are shown in FIG. 10. The tested anti-VEGFA/VEGFC bispecific antibodies could all inhibit the VEGFC-induced BaF3-FLT4 cell proliferation.

### Example 12. Assays for Inhibition of VEGFA + VEGFC-induced HUVEC Proliferation by Anti-VEGFA/VEGF C Bispecific Antibodies

VEGFA and VEGFC could act on VEGFR and other related receptors in vascular endothelial cells, and promote vascular endothelial cell survival, proliferation and migration, thereby inducing neovascularization. This assay utilized VEGFA and VEGFC to jointly induce the survival and proliferation of human umbilical vein endothelial cells (HUVECs), and the inhibitory effects of the antibodies on the VEGFA- and VEGFC-induced survival and proliferation of primary cells were detected. This example determined the survival and proliferation of HUVECs by CCK-8, and the specific method was as follows: cells were treated one day in advance and plated in a 96-well culture plate at 2000 cells/well; the plate was put into a 37 °C incubator with 5% carbon dioxide and incubated for 24 h. After the cells were attached to the wall, an experimental medium (DMEM medium) containing or not containing VEGF A with a final concentration of 5 ng/mL and VEGFC with a final concentration of 50 ng/mL and/or the gradiently diluted antibodies was prepared, the endothelial cell medium in a 96-well plate was replaced by the experimental medium, and the plate was put into a 37 °C incubator with 5% carbon dioxide and incubated for 72 h. Experimental grouping was as follows:
Blank group: DMEM medium;
VEGFA group: DMEM medium + 5 ng/mL VEGF A;
VEGFC group: DMEM medium + 50 ng/mL VEGF C;
VEGFA + VEGFC group: DMEM medium + 5 ng/mL VEGF A + 50 ng/mL VEGF C;
IgG group: DMEM medium + 5 ng/mL VEGFA + 50 ng/mL VEGF C + isotype control IgG;
IBI304 + OPT-302 group: DMEM medium + 5 ng/mL VEGF A + 50 ng/mL VEGF C + IBI304 + OPT-302 (IBI304 was combined with OPT-302 in a molar ratio of 1:1); and
bispecific antibody group: EGM-2 medium + 5 ng/mL VEGF A + 50 ng/mL VEGF C + bispecific antibody to be tested.

After the cells were incubated in the experimental medium, CCK-8 detection solution (Dojindo) was added at 10 µL/well, and the plate was put into the 37 °C incubator with 5% carbon dioxide and incubated for 12-24 h. The absorbance OD₄₅₀-OD₆₂₀ values were read by using a multimode microplate reader.

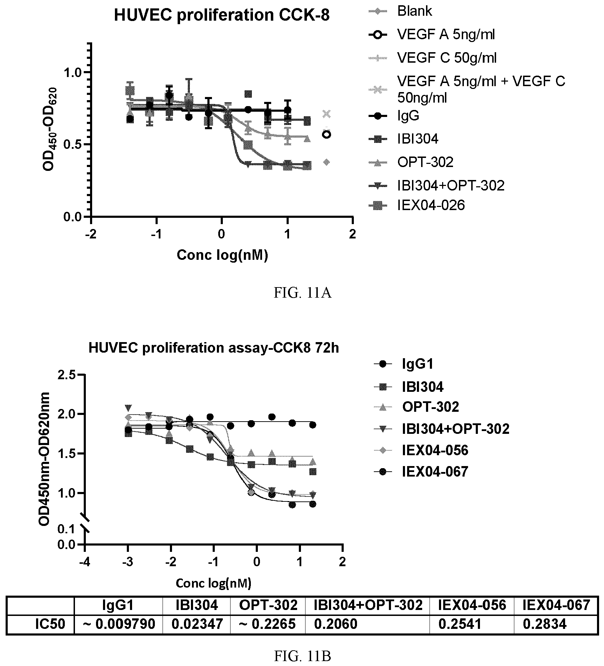

In the experiment described in the above assay, the results are shown in FIG. 11. The anti-VEGFA and anti-VEGFC bispecific antibodies tested were able to completely inhibit the VEGFA+VEGFC-induced proliferation and survival of HUVECs.

### Example 13. Assays for Inhibition of VEGFA + VEGFC-Induced HUVEC Tube Formation by Anti-VEGFA/VEGF C Bispecific Antibodies

VEGF A and VEGF C could act on vascular endothelial cells and promote the vascular endothelial cells to form a tube-like structure, thereby inducing the formation of a neovascular structure. This assay utilized VEGFA and VEGF C to induce the tube formation in human umbilical vein endothelial cells (HUVECs), and the inhibitory effects of the antibodies on the VEGF A- and VEGF C-induced tube formation in primary cells were detected.

This example detected the inhibition of HUVEC tube formation by the anti-VEGFA/VEGF C bispecific antibodies through HUVEC tube assays, and the specific method was as follows: matrigel (BD) was thawed on ice one day in advance, 100 µL of matrigel was added to a 96-well plate per well, and the plate was put into a 37 °C incubator with CO₂ for 0.5 h for curing. Cells were treated with an accutase solution and plated in the 96-well culture plate at 20000 cells/well, and the plate was put into the 37 °C incubator with 5% carbon dioxide and incubated for 24 h. Experimental media (EGM2) containing or not containing VEGF A with a final concentration of 10 ng/mL and VEGF C with a final concentration of 50 ng/mL and/or the gradiently diluted antibodies were prepared. HUVECs were resuspended in different experimental media and plated in the 96-well culture plate at 20000 cells/well, and the plate was put into the 37 °C incubator with 5% carbon dioxide and incubated for 24 h. Experimental grouping was as follows:
Blank group: EGM-2 medium;
VEGFA group: EGM-2 medium + 5 ng/mL VEGFA;
VEGFC group: EGM-2 medium + 50 ng/mL VEGFC;
VEGFA + VEGFC group: EGM-2 medium + 5 ng/mL VEGFA + 50 ng/mL VEGFC;
IgG group: EGM-2 medium + 5 ng/mL VEGFA + 50 ng/mL VEGFC + 20 nM isotype control IgG;
IBI304 + OPT-302 group: EGM-2 medium + 5 ng/mL VEGFA + 50 ng/mL VEGFC + 20 nM IBI304 + 20 nM OPT-302;
IEX04-056 group: EGM-2 medium + 5 ng/mL VEGFA + 50 ng/mL VEGFC + 20 nM IEX04-056; and
IEX04-067 group: EGM-2 medium + 5 ng/mL VEGFA + 50 ng/mL VEGFC + 20 nM IEX04-067.

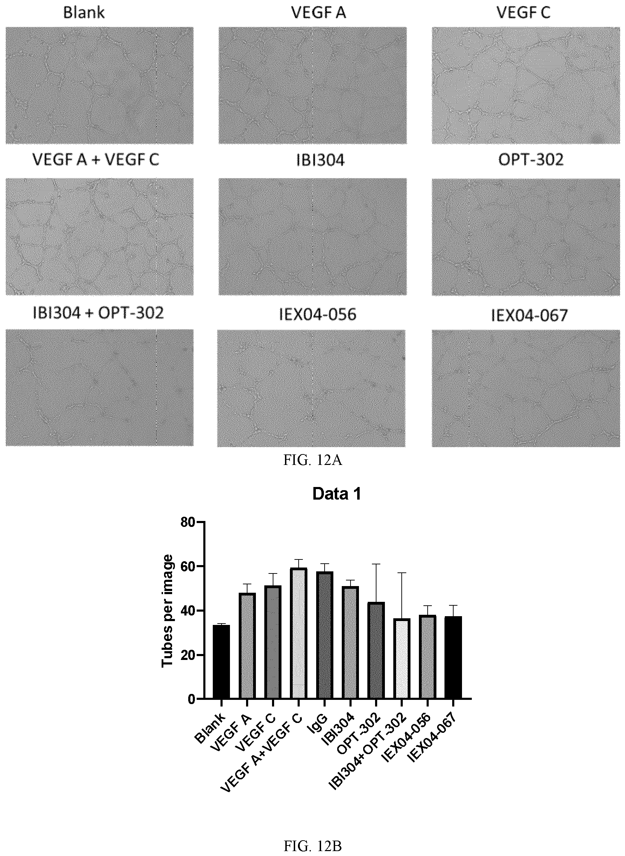

The number of the tubes was calculated by taking images with a microscope. The results are shown in FIG. 12. FIG. 12A shows tube formation images, and FIG. 12B shows statistical results of tube formation. The anti-VEGF A and anti-VEGF C bispecific antibodies were able to completely inhibit VEGF A + VEGF C-induced tube formation in HUVECs.

### Example 14. Assays for Inhibition of A375 Tumor Neovascularization by Anti-VEGFA/VEGFC Bispecific Antibodies

Overexpression of VEGFA and VEGFC in tumor cells could induce neovascularization *in vivo* and promote tumor growth.

This example determined the anti-neovascularization and anti-tumor effects of the anti-VEGFA/VEGF C antibodies of the present invention in nude mice by inoculating A375 human malignant melanoma cells at 3 × 10⁶ cells per mouse.

### Human-nude mice:

Female nude mice on the BALB/c background in an SPF grade were purchased from Beijing Vital River Laboratory Animal Technology Co., Ltd. The study started after the mice were acclimated for 7 days after arrival.

### Cells:

Human A375 cells were purchased from ATCC (CAT#: CRL-1619) and routinely subcultured strictly according to the instructions for subsequent *in vivo* assays. The cells were collected by centrifugation and resuspended in sterile PBS, with the cell density adjusted to 1.5×10⁷ cells/mL. On day 0, 0.2 mL of cell suspension was subcutaneously inoculated into the axilla of the mice to establish A375 tumor-bearing mouse models.

### Administration:

The mice were randomly grouped (6 mice in each group). 7 days and 21 days after the tumor cell inoculation, the tumor volume in each mouse was measured. The dose and route of administration are shown in Table 4. PBS (purchased from Gibco) was used as a negative control. The mice were administered on day 1, day 3, day 5, day 7, day 9, day 11, day 13, day 15, day 17, and day 19 after inoculation. The tumor volume and the body weight of the mice were monitored twice a week. The body weight and tumor volume were measured before each administration. On day 21 after inoculation, the relative tumor growth inhibition (TGI%) was calculated by the following formula: TGI% = 100% × (control group tumor volume - treatment group tumor volume)/(control group tumor volume - control group tumor volume before administration). Tumor volume measurement: The maximum length of major axis (L) and maximum length of minor axis (W) of tumors were measured with a vernier caliper, and tumor volume was calculated by the following formula: V = L × W²/2. The mice were weighted using an electronic balance.

**Table 4. Experimental design**

| Group | Dose of administration | Administration frequency | Route of administration |
|---|---|---|---|
| PBS | / | Q2DX10 | Intraperitoneal injection |
| IEX04-056 | 1 mg/kg | Q2DX10 | Intraperitoneal injection |
| IEX04-056 | 5 mg/kg | Q2DX10 | Intraperitoneal injection |
| IEX04-056 | 25 mg/kg | Q2DX10 | Intraperitoneal injection |

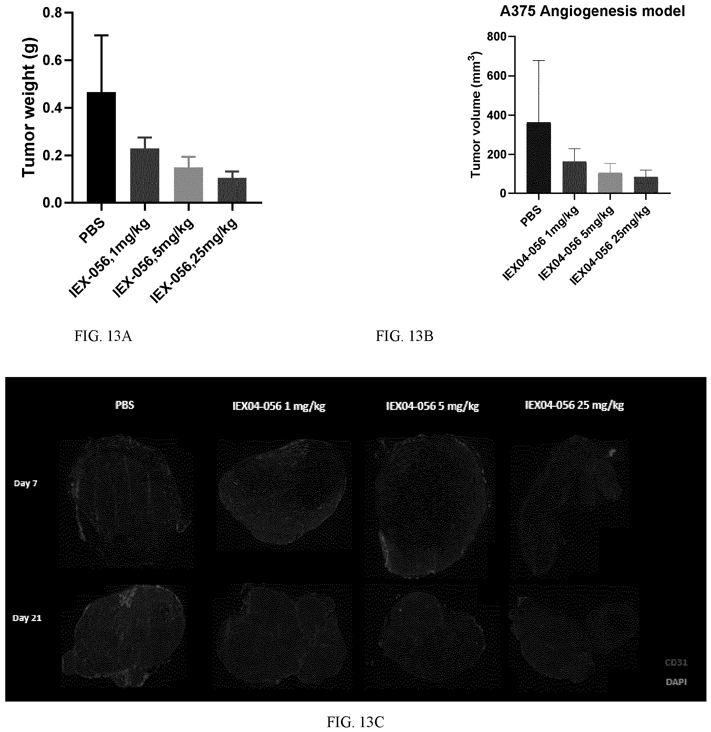

As shown in FIGs. 13A and 13B and Table 5, the results for the tumor growth inhibition showed that on day 21 after inoculation, IEX04-056, when used alone at different doses, showed a tumor inhibitory effect. On day 21 after inoculation, the tumor growth inhibition was 54% at 1 mg/kg, the tumor growth inhibition was 71% at 5 mg/kg, and the tumor growth inhibition was 74% at 25 mg/kg. Therefore, the bispecific binding molecules directed against VEGF A and VEGFC of the present invention had significant inhibitory effects on tumors.

**Table 5. Tumor growth inhibition on Day 21**

| Group | Mean tumor volume (mm³) | TGI(%) |
|---|---|---|
| PBS | 362 | / |
| IEX04-056 1 mg/kg | 163 | 54% |
| IEX04-056 5 mg/kg | 106 | 71% |
| IEX04-056 25 mg/kg | 84 | 77% |

Tumor tissues on day 7 and day 21 after inoculation were sectioned and stained with CD31 (vascular endothelial marker) to show microangiogenesis in the tumor tissues. A wax block embedded with an OCT embedding medium was placed in a cryostat, and the wax block was sliced into sections with a thickness of 6 µm.
1. The sections were left to stand at room temperature for 30 min, fixed with pre-cooled acetone at 4 °C, and dried in air at room temperature.
2. The sections were immersed and rinsed three times with PBST, once for 5 min (PBST: 1 × PBS + 0.05% Tween 20).
3. An immunohistochemistry pen was adopted to draw circles, and then the sections were then blocked with 10% goat serum (diluted with PBST) at room temperature, 100 µL goat serum for each tissue.
4. 100 µL of primary antibody PE-anti-mouse CD31 was directly added dropwise, and the sections were incubated at 4 °C overnight.
5. The next day, the sections were immersed and rinsed three times with PBST, once for 5 min.
6. Cell nuclei were stained with DAPI, and 100 µL of DAPI was added dropwise to each tissue. The sections were incubated in the dark for 5 min.
7. The DAPI staining solutions were discarded, and the sections were immersed and rinsed three times with PBST, once for 5 min.

The sections were mounted with a prolong diamond antifade mountant (Invitrogen), and then scanned using a fully automated quantitative analysis scanner. The results are shown in FIG. 13C. A375 tumor neovascularization was significantly reduced after IEX04-056 treatment.

### Example 15. Laser-Induced Choroidal Neovascularization Pharmacodynamic Test

This assay adopted a cynomolgus monkey laser-induced choroidal neovascularization model to determine the anti-neovascularization effect of the anti-VEGFA/VEGFC bispecific antibodies of the present invention.

Species: cynomolgus monkey; grade: normal; age: 2.5-5 years old; body weight: 2.45-5.55 kg, with an average body weight of 4.02 kg; body weight during modeling: 3.35-4.35 kg.

In this test, laser photocoagulation was performed around the macular fovea of the fundus of the cynomolgus monkey to induce the choroidal neovascularization in the fundus and establish an animal model similar to the human choroidal neovascularization. Before and 20 days after photocoagulation, fundus fluorescein angiography was performed to judge the molding condition. 16 cynomolgus monkeys (half male and half female) that were successfully molded were selected to be divided into 4 groups, that is, a model control group, an IEX04-056 group, an IEX04-067 group, and an Elyea + OPT-302 group, wherein each group contained 4 monkeys. On 21 days after photocoagulation, each group was administered individually according to the doses in Table 6 below. The IEX04-056 group, IEX04-067 group, or Eylea + OPT-302 group was administered by intravitreal injection at both eyes, and the model control group was given an equal volume of 0.9% sodium chloride injectable solution. Color fundus photography and fundus fluorescein angiography were performed on day 7, day 14, day 21, and day 28 after administration in each group of animals, and optical coherence tomography scanning was performed to observe the inhibition of the test samples on the choroidal neovascularization. The animals were euthanized on day 29 after administration, and then both eyes were taken out for HE staining histological examination.

**Table 6. Experimental design**

| Group | Dose | Administration volume | Concentration | Route of administration |
|---|---|---|---|---|
| Control | / | / | / | |
| IEX04-056 | 20 µg/eye | 50 µL/eye | 0.4 mg/mL | Intravitreal administration |
| IEX04-067 | 20 µg/eye | 50 µL/eye | 0.4 mg/mL | Intravitreal administration |
| Eylea+OPT-302 | 10 µg/eye + 10 µg/eye | 50 µL/eye | 0.4 mg/mL | Intravitreal administration |

### Color fundus photography and fluorescein angiography

### Evaluation indexes:

### (1) Fluorescent spot rating

Grading criteria of spots shot by fluorescence angiography after molding:
Grade 1: the spot has no high fluorescence;
Grade 2: the spot has high fluorescence but no fluorescein leakage;
Grade 3: the spot has high fluorescence and slight fluorescein leakage, and the leakage does not exceed the edge of the spot; and
Grade 4: the spot has high fluorescence and significant fluorescein leakage, and the leakage exceeds the edge of the spot.

Spots of grades 1-4 were counted, and the grade of the fundus laser spot needed to be recorded in each examination.

### (2) Improvement rate of fluorescein leakage area

Improvement rate of fluorescein leakage area (%) = (fluorescein leakage area before administration - fluorescein leakage area after administration)/fluorescein leakage area before administration × 100%

### (3) Reduction in fluorescein leakage area

Reduction in fluorescein leakage area = fluorescein leakage area before administration - fluorescein leakage area after administration

### Optical coherence tomography (OCT) scanning

### Evaluation indexes:

### 1) Improvement rate of fundus retinal thickening

Improvement rate of fundus retinal thickening (%) = retinal thickness before administration - retinal thickness after administration □ 100 Retinal thickness before administration - retinal thickness before modeling

### (2) Reduction in fundus retinal thickness

Reduction in fundus retinal thickness = retinal thickness before administration - retinal thickness after administration

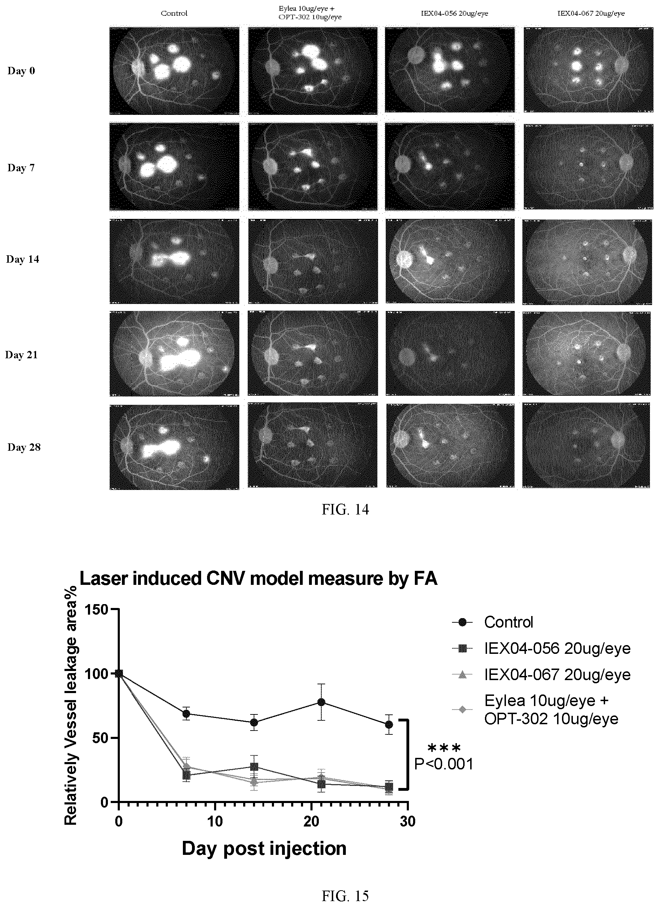

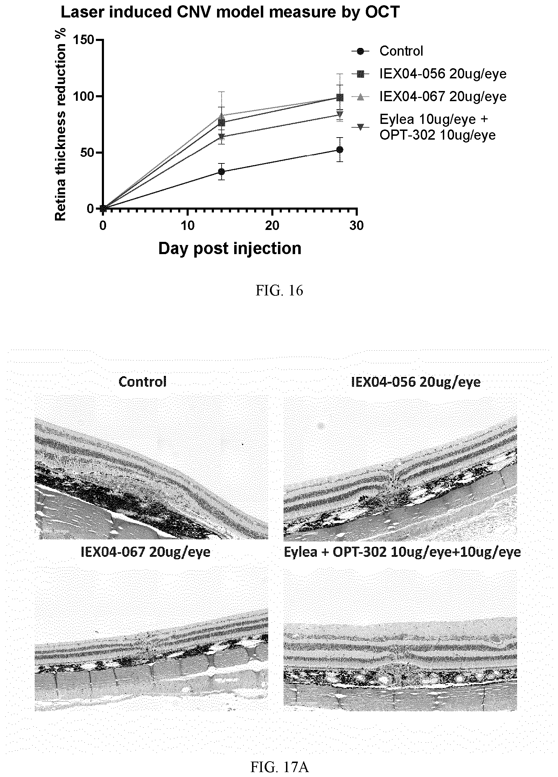

The results of color fundus photography and fluorescein angiography shown in FIGs. 14-15 show that the antibodies of the present invention showed significant anti-neovascularization effects 28 days after administration, which proves that the antibodies of the present invention have significant inhibitory effects (P < 0.001) on the laser-induced fundus neovascularization and have the function of protecting the integrity of blood vessels. The results of OCT shown in FIG. 16 show that the antibodies of the present invention significantly inhibit retinal thickening (P < 0.05) 14-28 days after administration, which proves that the antibodies of the present invention have the functions of inhibiting retinal edema and thickening caused by neovascularization.

### Histopathological examination

29 days after administration, cynomolgus monkeys were anesthetized with pentobarbital sodium according to the body weight (at about 30 mg/kg by intravenous injection, and the dose could be adjusted according to health conditions of the animals) and euthanized by bleeding from abdominal aorta or femoral artery. The cynomolgus monkeys were roughly observed, and bilateral eyeballs were taken.

Some animal eyes were fixed in a modified Davidson's fixative solution, the sections were embedded in paraffin, and laser-molded areas were selected for conventional HE staining and the like to perform the histopathological examination.

Compared with the combination of the anti-VEGFA molecule Eylea and the anti-VEGFC molecule OPT-032, the antibodies of the present invention were able to better improve the pathological changes such as edema, hyperplasia, and fibrosis of a laser-damaged site in the pathological section, showing better retinal morphology improvement (FIGs. 17A and 17B).

SEQUENCE LISTING
CDR1 sequence of LA49G9 antibody: SEQ ID NO: 1
GSMFSMYAMG
CDR2 sequence of LA49G9 antibody: SEQ ID NO: 2
ATTSGGSTLYADSVKG
CDR3 sequence of LA49G9 antibody: SEQ ID NO: 3
YWRGSDPENY
VHH sequence of LA49G9 antibody: SEQ ID NO: 4
QLQLVESGGGLVQPGGSLRLSCAASGSMFSMYAMGWYRQAPGKQRELVAATTSGGSTLYADSVKGRFTI
SRDNAENTVYLQMNSLKPEDTAVYYCYTYWRGSDPENYWGRGTQVTVSS

CDR1 sequence of LA49G9.2 antibody: SEQ ID NO: 5
GSMFSMYAMG
CDR2 sequence of LA49G9.2 antibody: SEQ ID NO: 6
ATTSGGSTLYADSVKG
CDR3 sequence of LA49G9.2 antibody: SEQ ID NO: 7
YWRGSDPENY
VHH sequence of LA49G9.2 antibody: SEQ ID NO: 8
EVQLVESGGGLVQPGGSLRLSCAASGSMFSMYAMGWYRQAPGKQRELVAATTSGGSTLYADSVKGRFTI
SRDNSKNTLYLQMNSLRAEDTAVYYCYTYWRGSDPENYWGQGTQVTVSS

CDR1 sequence of LA63G12 antibody: SEQ ID NO: 9
GSSFSPYAMG
CDR2 sequence of LA63G12 antibody: SEQ ID NO: 10
ATTSGGSTLYADSVKG
CDR3 sequence of LA63G12 antibody: SEQ ID NO: 11
HWRGSDPENY
VHH sequence of LA63G12 antibody: SEQ ID NO: 12
QLQLVESGGGLVQPGGSLRLSCAASGSSFSPYAMGWYRQAPGKQRELVAATTSGGSTLYADSVKGRFTIS
RDNAKNTVYLQMNSLKPEDTAVYYCYMHWRGSDPENYWGQGTQVTVSS

CDR1 sequence of LA63G12.1 antibody: SEQ ID NO: 13
GSSFSPYAMG
CDR2 sequence of LA63G12.1 antibody: SEQ ID NO: 14
ATTSGGSTLYADSVKG
CDR3 sequence of LA63G12.1 antibody: SEQ ID NO: 15
HWRGSDPENY
VHH sequence of LA63G12.1 antibody: SEQ ID NO: 16
EVQLVESGGGLVQPGGSLRLSCAASGSSFSPYAMGWYRQAPGKQRELVSATTSGGSTLYADSVKGRFTIS
RDNSKNTVYLQMNSLRAEDTAVYYCYMHWRGSDPENYWGQGTQVTVSS

CDR1 sequence of am63G12.1-14B11 antibody: SEQ ID NO: 17
GSSFSPYAMG
CDR1 sequence of am63G12.1-14B11 antibody: SEQ ID NO: 18
ATSEGGFTLYADSVKG

CDR1 sequence of am63G12.1-14B11 antibody: SEQ ID NO: 19
HWRGSDPENY
VHH sequence of am63G12.1-14B11 antibody: SEQ ID NO: 20
EVQLVESGGGLVQPGGSLRLSCAASGSSFSPYAMGWYRQAPGKQRELVSATSEGGFTLYADSVKGRFTIS
RDNSKNTVYLQMNSLRAEDTAVYYCYMHWRGSDPENYWGQGTQVTVSS

CDR1 sequence of am63G12.1-5G8-18B9 antibody: SEQ ID NO: 21
GSSFSPYAMG
CDR2 sequence of am63G12.1-5G8-18B9 antibody: SEQ ID NO: 22
ATSSGGFTLYADSVKG
CDR3 sequence of am63G12.1-5G8-18B9 antibody: SEQ ID NO: 23
YYREYDPEMY
VHH sequence of am63G12.1-5G8-18B9 antibody: SEQ ID NO: 24
EVQLVESGGGLVQPGGSLRLSCAASGSSFSPYAMGWYRQAPGKQRELVSATSSGGFTLYADSVKGRFTIS
RDNSKNTVYLQMNSLRAEDTAVYYCYTYYREYDPEMYWGQGTQVTVSS

Complete sequence of Aflibercept: SEQ ID NO: 25
SDTGRPFVEMYSEIPEIIHMTEGRELVIPCRVTSPNITVTLKKFPLDTLIPDGKRIIWDSRKGFIISNATYKEIG
LLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPA
PELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQK
SLSLSPG

Aflibercept VEGF-A binding domain: SEQ ID NO: 26
SDTGRPFVEMYSEIPEIIHMTEGRELVIPCRVTSPNITVTLKKFPLDTLIPDGKRIIWDSRKGFIISNATYKEIG
LLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEK

Aflibercept human IgG1 Fc region: SEQ ID NO: 27
DKTHTCPPCPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGNVFSCSVMHE
ALHNHYTQKSLSLSPG

Ranibizumab Fab VH-CH1: SEQ ID NO: 28
EVQLVESGGGLVQPGGSLRLSCAASGYDFTHYGMNWVRQAPGKGLEWVGWINTYTGEPTYAADFKRRF
TFSLDTSKSTAYLQMNSLRAEDTAVYYCAKYPYYYGTSHWYFDVWGQGTLVTVSSASTKGPSVFPLAPS
SKSTSGGTAALGCLVKDYFPEPVTVSWNSGALTSGVHTFPAVLQSSGLYSLSSVVTVPSSSLGTQTYICNV
NHKPSNTKVDKKVEPKSCDKTHL

Ranibizumab Fab VH: SEQ ID NO: 29



Ranibizumab Fab CH1: SEQ ID NO: 30
ASTKGPSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHL

Ranibizumab Fab VL-CL: SEQ ID NO: 31
DIQLTQSPSSLSASVGDRVTITCSASQDISNYLNWYQQKPGKAPKVLIYFTSSLHSGVPSRFSGSGSGTDFT
LTISSLQPEDFATYYCQQYSTVPWTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
C

Ranibizumab Fab VL: SEQ ID NO: 32



Ranibizumab Fab CL: SEQ ID NO: 33



Ranibizumab Fab: HCDR1 SEQ ID NO: 34
GYDFTHY
Ranibizumab Fab: HCDR2 SEQ ID NO: 35
NTYTGE
Ranibizumab Fab: HCDR3 SEQ ID NO: 36
YPYYYGTSHWYFDV
Ranibizumab Fab: LCDR1 SEQ ID NO: 37
QDISNYLN
Ranibizumab Fab: LCDR2 SEQ ID NO: 38
FTSSLHS
Ranibizumab Fab: LCDR3 SEQ ID NO: 39
QQYSTVPWT

IEX04-026     SEQ ID NO: 40
SDTGRPFVEMYSEIPEIIHMTEGRELVIPCRVTSPNITVTLKKFPLDTLIPDGKRIIWDSRKGFIISNATYKEIG
LLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPA
PELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQK
SLSLSPGGGGGSGGGGSGGGGSQLQLVESGGGLVQPGGSLRLSCAASGSSFSPYAMGWYRQAPGKQREL
VAATTSGGSTLYADSVKGRFTISRDNAKNTVYLQMNSLKPEDTAVYYCYMHWRGSDPENYWGQGTQVT
VSS

IEX04-037     SEQ ID NO: 41

SDTGRPFVEMYSEIPEIIHMTEGRELVIPCRVTSPNITVTLKKFPLDTLIPDGKRIIWDSRKGFIISNATYKEIG
LLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPA
PELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQK
SLSLSPGGGGGSGGGGSGGGGSEVQLVESGGGLVQPGGSLRLSCAASGSSFSPYAMGWYRQAPGKQREL
VSATTSGGSTLYADSVKGRFTISRDNSKNTVYLQMNSLRAEDTAVYYCYMHWRGSDPENYWGQGTQVT
VSS

IEX04-039 VH-CH1-linker-VHH SEQ ID NO: 42
EVQLVESGGGLVQPGGSLRLSCAASGYDFTHYGMNWVRQAPGKGLEWVGWINTYTGEPTYAADFKRRF
TFSLDTSKSTAYLQMNSLRAEDTAVYYCAKYPYYYGTSHWYFDVWGQGTLVTVSSASTKGPSVFPLAPS
SKSTSGGTAALGCLVKDYFPEPVTVSWNSGALTSGVHTFPAVLQSSGLYSLSSVVTVPSSSLGTQTYICNV
NHKPSNTKVDKKVEPKSCDKTHLGGGGSGGGGSGGGGSEVQLVESGGGLVQPGGSLRLSCAASGSSFSP
YAMGWYRQAPGKQRELVSATTSGGSTLYADSVKGRFTISRDNSKNTVYLQMNSLRAEDTAVYYCYMHW
RGSDPENYWGQGTQVTVSS

IEX04-039 VL-CL-linker-VHH SEQ ID NO: 43
DIQLTQSPSSLSASVGDRVTITCSASQDISNYLNWYQQKPGKAPKVLIYFTSSLHSGVPSRFSGSGSGTDFT
LTISSLQPEDFATYYCQQYSTVPWTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
CGGGGSGGGGSGGGGSEVQLVESGGGLVQPGGSLRLSCAASGSSFSPYAMGWYRQAPGKQRELVSATTS
GGSTLYADSVKGRFTISRDNSKNTVYLQMNSLRAEDTAVYYCYMHWRGSDPENYWGQGTQVTVSS

IEX04-041 SEQ ID NO: 44
SDTGRPFVEMYSEIPEIIHMTEGRELVIPCRVTSPNITVTLKKFPLDTLIPDGKRIIWDSRKGFIISNATYKEIG
LLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPA
PELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQK
SLSLSPGGGGGSGGGGSEVQLVESGGGLVQPGGSLRLSCAASGSSFSPYAMGWYRQAPGKQRELVSATTS
GGSTLYADSVKGRFTISRDNSKNTVYLQMNSLRAEDTAVYYCYMHWRGSDPENYWGQGTQVTVSS

IEX04-042 SEQ ID NO: 45
SDTGRPFVEMYSEIPEIIHMTEGRELVIPCRVTSPNITVTLKKFPLDTLIPDGKRIIWDSRKGFIISNATYKEIG
LLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPA
PELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQK
SLSLSPGGGGGSGGGGSGGGGSGGGGSEVQLVESGGGLVQPGGSLRLSCAASGSSFSPYAMGWYRQAPG
KQRELVSATTSGGSTLYADSVKGRFTISRDNSKNTVYLQMNSLRAEDTAVYYCYMHWRGSDPENYWGQ
GTQVTVSS

IEX04-046 SEQ ID NO: 46
SDTGRPFVEMYSEIPEIIHMTEGRELVIPCRVTSPNITVTLKKFPLDTLIPDGKRIIWDSRKGFIISNATYKEIG
LLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPA
PELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQK
SLSLSPGGGGGSGGGGSGGGGSEVQLVESGGGLVQPGGSLRLSCAASGSSFSPYAMGWYRQAPGKQREL
VSATSEGGFTLYADSVKGRFTISRDNSKNTVYLQMNSLRAEDTAVYYCYMHWRGSDPENYWGQGTQVT
VSS

IEX04-056 SEQ ID NO: 47
SDTGRPFVEMYSEIPEIIHMTEGRELVIPCRVTSPNITVTLKKFPLDTLIPDGKRIIWDSRKGFIISNATYKEIG
LLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPA
PELLGGPSVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEALHNHYTQK
SLSLSPGGGGGSGGGGSGGGGSEVQLVESGGGLVQPGGSLRLSCAASGSSFSPYAMGWYRQAPGKQREL
VSATSSGGFTLYADSVKGRFTISRDNSKNTVYLQMNSLRAEDTAVYYCYTYYREYDPEMYWGQGTQVT
VSS

IEX04-067 VH-CH1-linker-VHH SEQ ID NO: 48
EVQLVESGGGLVQPGGSLRLSCAASGYDFTHYGMNWVRQAPGKGLEWVGWINTYTGEPTYAADFKRRF
TFSLDTSKSTAYLQMNSLRAEDTAVYYCAKYPYYYGTSHWYFDVWGQGTLVTVSSASTKGPSVFPLAPS
SKSTSGGTAALGCLVKDYFPEPVTVSWNSGALTSGVHTFPAVLQSSGLYSLSSVVTVPSSSLGTQTYICNV
NHKPSNTKVDKKVEPKSCDKTHLGGGGSGGGGSGGGGSEVQLVESGGGLVQPGGSLRLSCAASGSSFSP
YAMGWYRQAPGKQRELVSATSEGGFTLYADSVKGRFTISRDNSKNTVYLQMNSLRAEDTAVYYCYMHW
RGSDPENYWGQGTQVTVSS

IEX04-067 VL-CL-linker-VHH SEQ ID NO: 49
DIQLTQSPSSLSASVGDRVTITCSASQDISNYLNWYQQKPGKAPKVLIYFTSSLHSGVPSRFSGSGSGTDFT
LTISSLQPEDFATYYCQQYSTVPWTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
CGGGGSGGGGSGGGGSEVQLVESGGGLVQPGGSLRLSCAASGSSFSPYAMGWYRQAPGKQRELVSATSE
GGFTLYADSVKGRFTISRDNSKNTVYLQMNSLRAEDTAVYYCYMHWRGSDPENYWGQGTQVTVSS

IEX04-069 VH-CH1-linker-VHH SEQ ID NO: 50
EVQLVESGGGLVQPGGSLRLSCAASGYDFTHYGMNWVRQAPGKGLEWVGWINTYTGEPTYAADFKRRF
TFSLDTSKSTAYLQMNSLRAEDTAVYYCAKYPYYYGTSHWYFDVWGQGTLVTVSSASTKGPSVFPLAPS
SKSTSGGTAALGCLVKDYFPEPVTVSWNSGALTSGVHTFPAVLQSSGLYSLSSVVTVPSSSLGTQTYICNV
NHKPSNTKVDKKVEPKSCDKTHLGGGGSGGGGSGGGGSEVQLVESGGGLVQPGGSLRLSCAASGSSFSP

YAMGWYRQAPGKQRELVSATSSGGFTLYADSVKGRFTISRDNSKNTVYLQMNSLRAEDTAVYYCYTYYR
EYDPEMYWGQGTQVTVSS

IEX04-069    VL-CL-linker-VHH SEQ ID NO: 51
DIQLTQSPSSLSASVGDRVTITCSASQDISNYLNWYQQKPGKAPKVLIYFTSSLHSGVPSRFSGSGSGTDFT
LTISSLQPEDFATYYCQQYSTVPWTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
CGGGGSGGGGSGGGGSEVQLVESGGGLVQPGGSLRLSCAASGSSFSPYAMGWYRQAPGKQRELVSATSS
GGFTLYADSVKGRFTISRDNSKNTVYLQMNSLRAEDTAVYYCYTYYREYDPEMYWGQGTQVTVSS

Positive control antibody anti-VEGF-C (VEGFR3-trap molecule) OPT-302       SEQ ID NO: 52
YSMTPPTLNITEESHVIDTGDSLSISCRGQHPLEWAWPGAQEAPATGDKDSEDTGVVRDCEGTDARPYCK
VLLLHEVHAQDTGSYVCYYKYIKARIEGTTAASSYVFVRDFEQPFINKPDTLLVNRKDAMWVPCLVSIPG
LNVTLRSQSSVLWPDGQEVVWDDRRGMLVSTPLLHDALYLQCETTWGDQDFLSNPFLVHITGNELYDIQ
LLPRKSLELLVGEKLVLNCTVWAEFNSGVTFDWDYPGKQAERGKWVPERRSQQTHTELSSILTIHNVSQH
DLGSYVCKANNGIQRFRESTEVIVHEEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK

Sequence of IBI304 molecule: SEQ ID NO: 53
PFVEMYSEIPEIIHMTEGRELVIPCRVTSPNITVTLKKFPLDTLIPDGKRIIWDSRKGFIISNATYKEIGLLTCE
ATVNGHLYKTNYLTHRQTVVLSPSHGIELSVGEKLVLNCTARTELNVGIDFNWEYPSSKHQHKKLVNRDL
KTQSGSEMKKFLSTLTIDGVTRSDQGLYTCAASSGLMTKKNSTFVRVHEKGGGGGGGGGGGDKTHTC
PLCPAPELLGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRDELTKNQVSLT
CLVKGFYPSDIAVEWESNGQPENNYKATPPVLDSDGSFFLYSKLTVDKSRWQQGNVFSCSVMHEALHNH
YTQKSLSLSPGK

## Claims

1. A single-domain antibody (sdAb) polypeptide specifically binding to human VEGF C, comprising a VHH domain of the following formula consisting of 3 CDRs and 4 FRs:
FR1-CDR1-FR2-CDR2-FR3 -CDR3 -FR4;
wherein the VHH domain comprises:
(i) CDR1 comprising or consisting of an amino acid sequence set forth in SEQ ID NO: 1, CDR2 comprising or consisting of an amino acid sequence set forth in SEQ ID NO: 2, and CDR3 comprising or consisting of an amino acid sequence set forth in SEQ ID NO: 3;
(ii) CDR1 comprising or consisting of an amino acid sequence set forth in SEQ ID NO: 9, CDR2 comprising or consisting of an amino acid sequence set forth in SEQ ID NO: 10, and CDR3 comprising or consisting of an amino acid sequence set forth in SEQ ID NO: 11;
(iii) CDR1 comprising or consisting of an amino acid sequence set forth in SEQ ID NO: 17, CDR2 comprising or consisting of an amino acid sequence set forth in SEQ ID NO: 18, and CDR3 comprising or consisting of an amino acid sequence set forth in SEQ ID NO: 19; or
(iv) CDR1 comprising or consisting of an amino acid sequence set forth in SEQ ID NO: 21, CDR2 comprising or consisting of an amino acid sequence set forth in SEQ ID NO: 22, and CDR3 comprising or consisting of an amino acid sequence set forth in SEQ ID NO: 23.

2. The polypeptide according to claim 1, wherein the VHH domain comprises:
(i) an amino acid sequence selected from SEQ ID NOs: 4, 8, 12, 16, 20, and 24, preferably an amino acid sequence set forth in SEQ ID NO: 20, and more preferably an amino acid sequence set forth in SEQ ID NO: 24; or
(ii) an amino acid sequence having at least 80%, 85%, 90%, 91%, 92%, 93%, 94%, 95%, 96%, 97%, 98%, 99%, or more identity to the amino acid sequence of (i); or
(iii) an amino acid sequence having at least 1-30, 1-20, 1-15, 1-10, or 1-5 amino acid changes (e.g., substitutions, deletions, and/or insertions, preferably substitutions, and more preferably conservative substitutions) relative to the amino acid sequence of (i).

3. The polypeptide according to claim 1, wherein the VHH domain is humanized.

4. The polypeptide according to claim 1, wherein the single-domain antibody polypeptide is a single-chain antibody polypeptide consisting of the VHH domain.

5. A protein comprising at least one single-domain antibody polypeptide according to claim 1, wherein the protein is, for example, a fusion protein or a chimeric polypeptide, preferably a VHH-Fc antibody.

6. A bispecific binding protein, comprising
(i) a first antigen-binding component specifically binding to human VEGF C; and
(ii) a second antigen-binding component specifically binding to human VEGF A,
wherein the first antigen-binding component comprises the single-domain antibody polypeptide according to any one of claims 1-4; and
the bispecific binding protein inhibits the binding of VEGF A to its VEGF receptor and inhibits the binding of VEGF C to its VEGF receptor.

7. The bispecific binding protein according to claim 6, wherein the first antigen-binding component is linked to the second antigen-binding component via a linker; preferably, the linker comprises an amino acid sequence G(G4S)n or (G4S)n, and n is an integer of 1, 2, 3, 4, or 5, preferably n = 2, 3, or 4.

8. The bispecific binding protein according to claim 6 or 7, wherein the second antigen-binding component is selected from an anti-VEGF A antibody (e.g., a single-chain Fv antibody, a Fab antibody, a Fab' antibody, a (Fab)₂ antibody, a single-domain antibody, and a nanobody), a VEGF-A Trap molecule, or an Fc fusion protein comprising a VEGFA binding domain.

9. The bispecific binding protein according to claim 8, wherein the second antigen-binding component is the Fc fusion protein comprising the VEGF-A binding domain, preferably comprising a VEGF-A binding domain fused to the N-terminus of a human IgG Fc, such as a VEGF-A binding domain from VEGFR1 and/or VEGFR2 receptors; preferably, the VEGF-A binding domain comprises an amino acid sequence set forth in SEQ ID NO: 26, or an amino acid sequence having at least 85%, 90%, 95%, 96%, 97%, 98%, or 99% sequence identity thereto;
more preferably, a VEGF-A binding domain-Fc fusion polypeptide comprises an amino acid sequence set forth in SEQ ID NO: 25, or an amino acid sequence having at least 85%, 90%, 95%, 96%, 97%, 98%, or 99% sequence identity thereto.

10. The bispecific binding protein according to claim 9, wherein the single-domain antibody polypeptide binding to VEGF A is linked to the C-terminus of the Fc fusion polypeptide.

11. The bispecific binding protein according to claim 10, wherein the protein comprises a first polypeptide chain and a second polypeptide chain, wherein
the first polypeptide chain and the second polypeptide chain are identical and each comprises an amino acid sequence selected from SEQ ID NOs: 40-41 and 44-47, or an amino acid sequence having at least 85%, 90%, 95%, 96%, 97%, 98%, or 99% sequence identity thereto;
preferably, the first polypeptide chain and the second polypeptide chain are identical and each comprises an amino acid sequence set forth in SEQ ID NO: 47.

12. The bispecific binding protein according to claim 6 or 7, wherein the second antigen-binding component comprises an anti-VEGF-A Fab antibody consisting of VH-CH1 and VL-CL;
preferably, the VH comprises amino acid sequences of HCDR1-3 set forth in SEQ ID NOs: 34-36, and the VL comprises amino acid sequences of LCDR1-3 set forth in SEQ ID NOs: 37-39;
more preferably, the VH comprises an amino acid sequence set forth in SEQ ID NO: 29, and the VL comprises an amino acid sequence set forth in SEQ ID NO: 32;
still more preferably, a Fab fragment comprises amino acid sequences set forth in SEQ ID NO: 28 and SEQ ID NO: 31.

13. The bispecific binding protein according to claim 12, wherein the single-domain antibody polypeptide is linked, preferably linked via a linker, to the C-terminus of the VH-CH1 and/or VL-CL of the Fab antibody.

14. The bispecific binding protein according to claim 13, wherein the protein comprises a first polypeptide chain and a second polypeptide chain, wherein
the first polypeptide chain comprises the single-domain antibody polypeptide fused to the C-terminus of the VH-CH1 polypeptide of the Fab antibody; and
the second polypeptide chain comprises the single-domain antibody polypeptide fused to the C-terminus of the VL-CL polypeptide of the Fab antibody.

15. The bispecific binding protein according to claim 14, wherein the first polypeptide chain and the second polypeptide chain are selected from:
- a first polypeptide chain comprising an amino acid sequence set forth in SEQ ID NO: 42, or an amino acid sequence having at least 85%, 90%, 95%, 96%, 97%, 98%, or 99% sequence identity thereto, and a second polypeptide chain comprising an amino acid sequence set forth in SEQ ID NO: 43, or an amino acid sequence having at least 85%, 90%, 95%, 96%, 97%, 98%, or 99% sequence identity thereto;
- a first polypeptide chain comprising an amino acid sequence set forth in SEQ ID NO: 48, or an amino acid sequence having at least 85%, 90%, 95%, 96%, 97%, 98%, or 99% sequence identity thereto, and a second polypeptide chain comprising an amino acid sequence set forth in SEQ ID NO: 49, or an amino acid sequence having at least 85%, 90%, 95%, 96%, 97%, 98%, or 99% sequence identity thereto; and
- a first polypeptide chain comprising an amino acid sequence set forth in SEQ ID NO: 50, or an amino acid sequence having at least 85%, 90%, 95%, 96%, 97%, 98%, or 99% sequence identity thereto, and a second polypeptide chain comprising an amino acid sequence set forth in SEQ ID NO: 51, or an amino acid sequence having at least 85%, 90%, 95%, 96%, 97%, 98%, or 99% sequence identity thereto;
preferably, the first polypeptide chain comprises the amino acid sequence set forth in SEQ ID NO: 48, and the second polypeptide chain comprises the amino acid sequence set forth in SEQ ID NO: 49.

16. A polynucleotide encoding the single-domain antibody polypeptide according to claims 1-4, the protein according to claim 5, or the bispecific binding protein according to claims 6-15.

17. An expression vector comprising the polynucleotide according to claim 16.

18. A host cell transfected with the vector according to claim 17.

19. A method for producing the single-domain antibody polypeptide according to claims 1-4, the protein according to claim 5, or the bispecific binding protein according to claims 6-15, comprising culturing the host cell according to claim 18 and recovering the produced single-domain antibody polypeptide or bispecific binding protein.

20. A pharmaceutical composition comprising the single-domain antibody polypeptide according to claims 1-4, the protein according to claim 5, or the bispecific binding protein according to claims 6-15, and a pharmaceutically acceptable carrier.

21. A method for treating a neovascularization-associated disease, comprising administering to a subject the single-domain antibody polypeptide according to claims 1-4, the protein according to claim 5, or the bispecific binding protein according to claims 6-15, or a pharmaceutical composition thereof.

22. The method according to claim 21, wherein the disease is a solid tumor, preferably melanoma, and administration of the bispecific binding protein inhibits neovascularization in the tumor and/or tumor growth.

23. The method according to claim 21, wherein the disease is an ocular disease, preferably age-related macular degeneration, diabetic retinopathy, retinal vascular occlusion, and corneal neovascularization.

24. Use of the single-domain antibody polypeptide according to claims 1-4, the protein according to claim 5, or the bispecific binding protein according to claims 6-15 in the manufacture of a medicament for the treatment and/or prevention of a disease in a subject and/or in the manufacture of a diagnostic tool for the diagnosis of a disease, wherein the disease is preferably a neovascularization-associated disease, such as a solid tumor and an ocular disease.
